(19) Europäisches Patentamt
European Patent Office
Office européen des brevets

(11) **EP 3 665 478 B1**

(12) **FASCICULE DE BREVET EUROPEEN**

(45) Date de publication et mention
de la délivrance du brevet:
**16.06.2021 Bulletin 2021/24**

(51) Int Cl.:
**G01N 33/00** (2006.01)    **G01N 21/55** (2014.01)
**G01N 21/552** (2014.01)    **G01N 21/77** (2006.01)

(21) Numéro de dépôt: **18780183.2**

(22) Date de dépôt: **11.09.2018**

(86) Numéro de dépôt international:
**PCT/FR2018/052219**

(87) Numéro de publication internationale:
**WO 2019/053366 (21.03.2019 Gazette 2019/12)**

(54) **SYSTÈME DE DÉTECTION PERFECTIONNÉ POUR NEZ ÉLECTRONIQUE ET NEZ ÉLECTRONIQUE COMPRENANT UN TEL SYSTÈME**

VERBESSERTES DETEKTIONSSYSTEM FÜR EINE ELEKTRONISCHE NASE UND EINE ELEKTRONISCHE NASE MIT EINEM SOLCHEN SYSTEM

IMPROVED DETECTION SYSTEM FOR AN ELECTRONIC NOSE AND AN ELECTRONIC NOSE COMPRISING SUCH A SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **14.09.2017 FR 1758547**

(43) Date de publication de la demande:
**17.06.2020 Bulletin 2020/25**

(73) Titulaires:
• **Aryballe Technologies
38040 Grenoble Cedex 09 (FR)**
• **Commissariat à l'énergie atomique
et aux énergies alternatives
75015 Paris (FR)**
• **Université Grenoble Alpes
38400 Saint-Martin-d'Hères (FR)**
• **Centre National de la Recherche Scientifique
75794 Paris Cedex 16 (FR)**

(72) Inventeurs:
• **HOU-BROUTIN, Yanxia
38850 Bilieu (FR)**
• **BRENET, Sophie
38000 Grenoble (FR)**
• **LIVACHE, Thierry
38560 Jarrie (FR)**
• **HERRIER, Cyril
38000 Grenoble (FR)**
• **ROUSSELLE, Tristan
38100 Grenoble (FR)**

(74) Mandataire: **Brevalex
95, rue d'Amsterdam
75378 Paris Cedex 8 (FR)**

(56) Documents cités:
**EP-A1- 3 185 011**

• **BRENET SOPHIE ET AL: "Development of a novel multiplexed optoelectronic nose for analysis of volatile organic compounds", 2017 ISOCS/IEEE INTERNATIONAL SYMPOSIUM ON OLFACTION AND ELECTRONIC NOSE (ISOEN), IEEE, 28 mai 2017 (2017-05-28), pages 1-3, XP033114172, DOI: 10.1109/ISOEN.2017.7968845**
• **LEE Y-C ET AL: "Design and implementation of gas sensor array based on fluorescence quenching detection using CMOS-MEMS process", 2017 19TH INTERNATIONAL CONFERENCE ON SOLID-STATE SENSORS, ACTUATORS AND MICROSYSTEMS (TRANSDUCERS), IEEE, 18 juin 2017 (2017-06-18), pages 672-675, XP033130798, DOI: 10.1109/TRANSDUCERS.2017.7994138**

• LANG H P ET AL: "TUTORIAL; Nanomechanics from atomic resolution to molecular recognition based on atomic force microscopy technology; Nanomechanics from atomic resolution to molecular recognition based on atomic force microscopy technology", NANOTECHNOLOGY, IOP, BRISTOL, GB, vol. 13, no. 5, 1 octobre 2002 (2002-10-01), pages R29-R36, XP020067004, ISSN: 0957-4484, DOI: 10.1088/0957-4484/13/5/202

## Description

## DOMAINE TECHNIQUE

L'invention relève du domaine des nez électroniques.

Plus spécifiquement, l'invention se rapporte à un système de détection perfectionné pour nez électronique ainsi qu'un nez électronique comprenant un tel système de détection.

L'invention a de nombreuses applications et, notamment, dans la protection de l'environnement, en particulier pour la surveillance de la pollution olfactive et de la qualité d'ambiances plus ou moins confinées, dans la surveillance de sites fabriquant, stockant, manipulant et/ou susceptibles d'être contaminés par des matières volatiles potentiellement dangereuses ou odorantes, dans la santé, par exemple pour offrir un substitut à l'odorat à des personnes souffrant d'une anosmie ou pour détecter des marqueurs biologiques volatils, dans l'agro-alimentaire, par exemple pour la détection de contaminations dans une chaîne de fabrication et/ou de distribution de produits alimentaires, ainsi que pour le contrôle de tout produit ayant une odeur.

## ÉTAT DE LA TECHNIQUE ANTÉRIEURE

On appelle « nez électronique » un appareil permettant de détecter et d'identifier des odeurs et, donc, des composés cibles en phase gazeuse.

Le nez électronique doit son nom à l'analogie qui existe entre son fonctionnement et celui du système olfactif humain.

Un nez électronique se compose principalement de trois systèmes, à savoir :

(1) un système d'échantillonnage de la phase gazeuse à analyser;

(2) un système de détection qui comprend un réseau de capteurs aptes à interagir de façon physico-chimique avec les composés cibles, les capteurs jouant le rôle des récepteurs olfactifs ;

(3) un système informatique de traitement et d'analyse des réponses émises sous forme de signaux par capteurs sous forme de signaux suite à une interaction physico-chimique, ce système jouant le rôle du cerveau.

À titre d'exemple de nez électronique, on peut mentionner celui qui a été présenté par S. Brenet et al. au 17ème Symposium International sur l'Olfaction et les Nez électroniques (28-31 mai 2017, Montréal, Canada) et qui est décrit dans 2017 SOCS/IEEE International Symposium on Olfaction and Electronic Nose (ISOEN), IEEE, 2017, pp. 1-3, ci-après référence [1]. Ce nez électronique est conçu pour détecter et identifier des composés organiques volatils et comprend un système de détection optique par imagerie de résonance plasmonique de surface (ou SPRi).

Comme tous les appareils d'analyse dont le fonctionnement est basé sur l'émission de signaux par des capteurs, les nez électroniques sont le siège, d'une part, de signaux parasites que l'on regroupe sous le nom de « bruit de mesure », qui viennent se superposer aux signaux utiles, c'est-à-dire aux informations que l'on cherche à récupérer, et, d'autre part, à une dérive des capteurs.

Le bruit de mesure peut être causé par une multitude de facteurs externes ou internes au nez électronique comme des variations de température, de pression, d'humidité et, notamment, par des fluctuations du système de mesure liées à des variations des facteurs précités au cours de la mesure. Ce bruit peut survenir à tout moment et amener à des erreurs d'interprétation des signaux émis par les capteurs.

La dérive des capteurs consiste, elle, en une variation graduelle sur le long terme des signaux émis par les capteurs qui est observée alors que les capteurs sont mis en présence des mêmes composés cibles et dans les mêmes conditions opératoires. Elle est induite par des mécanismes physicochimiques complexes : elle pourrait notamment être due à un empoisonnement des capteurs ou à leur vieillissement mais elle pourrait aussi être due à des changements de paramètres physiques, environnementaux (tels que la température, la pression et l'humidité) et/ou expérimentaux (tels qu'un phénomène de chauffe des composants du nez électronique).

Dans les biocapteurs ou biopuces, dont le fonctionnement est basé sur le principe de clé-serrure, c'est-à-dire qu'une molécule biologiquement active (par exemple, un antigène) est reconnue par un ligand spécifique (par exemple, un anticorps spécifique de cet antigène), on utilise généralement des ligands non spécifiques des molécules à analyser comme contrôles négatifs afin de déterminer le bruit de mesure et toute dérive des capteurs et, ainsi, valider les signaux émis par ces capteurs.

De manière similaire, dans les multicapteurs chimiques de gaz dont le fonctionnement est basé sur une interaction entre des gaz et des capteurs spécifiques à un gaz, il est connu de prévoir la présence d'un capteur de référence pour compenser le bruit de mesure, ce capteur étant typiquement une zone du multicapteur qui est dépourvue de matériau sensible comme décrit, par exemple, par Y. C. Lee et al. (19th International Conference on Solid-State Sensors, Actuators and Microsystems (Transducers), IEEE, 2017, pp. 672-675, ci-après référence [2]) pour mesurer une référence de fluorescence (insensible à l'environnement chimique). Dans ce cas, les composés gazeux vont quencher le signal de fluorescence des capteurs.

En revanche, dans les nez électroniques, dont le fonctionnement est basé sur le principe d'une réactivité croisée, c'est-à-dire que chaque ligand, appelé récepteur, interagit avec plus ou moins d'affinité avec les composés cibles, tous les signaux émis par les capteurs sont pris en compte, qu'ils soient forts ou faibles, et c'est l'ensemble de ces signaux qui constitue un motif de reconnaissance (ou « recognition pattern » en anglais), ce motif étant caractéristique d'un composé cible et pouvant être considéré comme une empreinte digitale de ce composé (cf. référence **[1]**).

Ainsi, dans le cas des nez électroniques, il est actuellement impossible de déterminer si la faiblesse d'un signal émis par un capteur correspond à une absence d'affinité de ce capteur pour un ou plusieurs composés cibles ou à du bruit de mesure.

Pour déterminer une potentielle dérive des capteurs, il a été proposé d'utiliser un ou plusieurs gaz de référence (cf. Handbook of Machine Olfaction: Electronic Nose Technology, chapitre 13, John Wiley & Sons 2006, ci-après référence **[3]).** L'idée est d'effectuer des mesures avec le ou les gaz de référence au début de la série de mesures, puis à certains intervalles de temps aussi longtemps que les capteurs sont utilisés. Le changement de réponse des capteurs au(x) gaz de référence est pris comme mesure du changement de réponse pour toutes les autres mesures. Par conséquent, pour obtenir une bonne estimation de la dérive pour des échantillons gazeux à analyser, il convient de trouver un gaz de référence qui reflète parfaitement la dérive susceptible d'être obtenue avec ces échantillons. Or, il est très difficile, voire impossible de trouver un tel gaz de référence.

Par ailleurs, de nombreuses méthodes ont été développées pour réduire les effets de la dérive. Elles sont souvent basées sur des modèles mathématiques pour compenser les changements de performance des capteurs. Ces méthodes peuvent être réparties en quatre catégories principales : celles mettant en jeu un prétraitement des signaux émis par les capteurs, une calibration périodique, des méthodes d'harmonisation des données et des méthodes adaptatives (cf. S. Di Carlo et M. Falasconi, Drift correction methods for gas chemical sensors in artificial olfaction systems: techniques and challenges. InTech: 2012, ci-après référence **[4]**). Si certaines de ces méthodes comme les méthodes adaptatives (réseaux de neurones, algorithmes évolutionnistes, ...) semblent prometteuses, elles restent à ce jour difficilement utilisables en pratique.

Il résulte de ce qui précède que parvenir à déterminer le bruit de mesure et/ou une dérive des capteurs d'un nez électronique et, si dérive il y a, réduire les effets de cette dérive reste un problème majeur sachant que l'existence d'un bruit élevé et/ou d'une dérive des capteurs peut nuire à l'utilisation de ce type d'appareil pour un manque de fiabilité et de reproductibilité.

L'invention vise justement à fournir une solution à ce problème.

## EXPOSÉ DE L'INVENTION

L'invention a donc, en premier lieu, pour objet un système de détection d'un nez électronique apte à détecter et identifier un ensemble E de composés susceptibles d'être présents dans un échantillon gazeux, lequel système de détection comprend une pluralité de capteurs de détection à réactivité croisée pour fournir des signaux représentatifs de la présence d'un ou plusieurs composés de l'ensemble E dans l'échantillon gazeux, chaque capteur de détection comprenant une partie sensible, et est caractérisé en ce qu'il comprend :

- au moins un capteur de référence pour fournir un signal représentatif du bruit de mesure du système de détection, le capteur de référence comprenant une partie sensible ;
- un substrat comprenant une surface sur laquelle sont disposées les parties sensibles des capteurs de détection et la partie sensible du capteur de référence ;

et en ce que la surface du substrat comprend :

* une pluralité de zones sensibles, chacune de ces zones sensibles correspondant à la partie sensible de l'un des capteurs de détection et comprenant au moins un récepteur capable d'interagir physico-chimiquement avec au moins un composé de l'ensemble E ; et
* au moins une zone sensible qui correspond à la partie sensible du capteur de référence et qui est fonctionnalisée avec au moins un composé fluoré choisi parmi les composés comprenant au moins un groupe alkyle terminal perfluoré et les polymères fluorés.

Ainsi, selon l'invention, il est proposé de prévoir, dans un système de détection d'un nez électronique, la présence d'au moins un capteur (c'est-à-dire d'un ou plusieurs capteurs) dont la fonction n'est pas de fournir des informations sur les composés susceptibles d'être présents dans l'échantillon gazeux mais de donner des informations sur le bruit de mesure de ce système de détection et, par conséquent, de permettre une correction d'éventuelles dérives du bruit de mesure et/ou des capteurs de détection.

Il est de plus proposé que la partie sensible du ou des capteurs de référence soit fonctionnalisée avec au moins un composé fluoré, ce composé étant choisi parmi les composés comprenant au moins un groupe alkyle terminal perfluoré, c'est-à-dire au moins un groupe $-CF_3$, et les polymères fluorés, ce type de composés et de polymères ayant l'avantage de présenter à la fois une inertie chimique et des propriétés non-mouillantes.

Dans ce qui précède et ce qui suit, on entend :

* par « capteur », un ensemble qui comprend une partie sensible qui comprend au moins un récepteur capable d'interagir de façon physico-chimique avec au moins l'un des composés de l'ensemble E, et un système de mesure, typiquement appelé transducteur, dont la fonction est de mesurer une variation d'une grandeur physique résultant de l'interaction physico-chimique et de convertir cette mesure en un signal exploitable, étant entendu que chacun des capteurs du système de détection peut comprendre son propre système de mesure ou partager avec d'autres capteurs un système de mesure qui leur est commun ;

* par « réactivité croisée », le fait que la partie sensible d'un capteur de détection puisse interagir de façon physico-chimique avec différents composés de l'ensemble E et qu'inversement un composé de l'ensemble E puisse interagir de façon physico-chimique avec la partie sensible de différents capteurs de détection ;

* par « bruit de mesure », la partie d'un signal émis par un capteur qui est induite par des facteurs autres que son interaction physico-chimique avec l'un des composés de l'ensemble E, par exemple une variation de température, une variation de pression, une variation d'humidité, une variation de la tension d'alimentation électrique du système de détection, des vibrations, etc. ;

* par « dérive du système de mesure », une variation progressive dans le temps du niveau moyen du bruit de mesure ; et

* par « dérive d'un capteur », une variation progressive dans le temps du signal émis par un capteur par rapport au signal que ce capteur est censé émettre dans les mêmes conditions, c'est-à-dire lorsqu'il est exposé au même échantillon gazeux et avec les mêmes paramètres opératoires ; la dérive d'un capteur correspond à la somme de la dérive du système de mesure et de la dérive chimique du capteur, c'est-à-dire une variation progressive dans le temps de la capacité de la partie sensible de ce capteur à interagir de façon physico-chimique avec au moins l'un des composés de l'ensemble E.

Par ailleurs, on entend par « récepteur », toute molécule chimique, simple ou complexe (c'est-à-dire pouvant, notamment, être une macromolécule), qui, par elle-même ou lorsqu'elle est associée à un ou plusieurs autres récepteurs au sein d'un mélange de récepteurs, est apte à interagir de façon physico-chimique avec un ou plusieurs composés de l'ensemble E, cette interaction physico-chimique résidant typiquement dans une sorption et, plus spécifiquement, une adsorption.

De préférence, le composé fluoré est choisi parmi les composés de formule $C_vF_{2v+2}$ dans laquelle v est un entier allant de 4 à 20, et les composés de formule $C_wF_{2w+1}\text{-}(L)_x\text{-}Z$ dans laquelle w est un entier allant de 1 à 12, x vaut 0 ou 1, L représente un groupe divalent espaceur tandis que Z représente un groupe propre à permettre la fixation, par liaisons covalentes ou non, du composé sur la surface du substrat.

Le groupe divalent espaceur peut notamment être un groupe hydrocarboné linéaire ou ramifié, saturé ou insaturé, comprenant de 1 à 20 atomes de carbone et éventuellement un ou plusieurs hétéroatomes, cet ou ces hétéroatomes étant typiquement choisi(s) parmi l'oxygène, l'azote, le soufre et le silicium. Ainsi, le groupe divalent espaceur est, par exemple, un groupe alkylène divalent comprenant de 1 à 20 atomes de carbone et, mieux encore, de 1 à 12 atomes de carbone.

Avantageusement, Z représente un groupe thiol, amine, silanol, carbonyle ou carboxyle.

Selon une disposition particulièrement préférée de l'invention, le composé fluoré est un perfluoralcanethiol de formule $CF_3(CF_2)_y(CH_2)_zSH$ dans laquelle y est un nombre entier allant de 0 à 11 et z est un nombre entier allant de 1 à 20 et, de préférence, de 1 à 12.

À titre d'exemples d'un tel composé fluoré, on peut citer le 1*H*,1*H*-trifluoroéthanethiol, le 1*H*,1*H*,2*H*,2*H*-perfluoropentanethiol, le 1*H*,1*H*,2*H*,2*H*-perfluorohexanethiol, le 1*H*,1*H*,2*H*,2*H*-perfluorooctanethiol et le 1*H*,1*H*,2*H*,2*H*-perfluoro-décanethiol.

Comme précédemment indiqué, le composé fluoré peut également être un polymère fluoré, auquel cas il est avantageusement choisi parmi les polytétrafluoroéthylènes, les fluorures de polyvinyle, les fluorures de polyvinylidène, les perfluoroalcoxy alcanes, les copolymères d'éthylène et de propylène fluorés et les poly(éthylène-*co*-tétrafluoroéthylène).

Conformément à l'invention, la zone sensible correspondant à la partie sensible du capteur de référence peut être formée par l'une quelconque des techniques de fonctionnalisation de surfaces connues de l'homme du métier telles que l'adsorption physique ou chimique, le greffage covalent, l'épitaxie à jet moléculaire, le dépôt de couches minces, l'auto-assemblage moléculaire, etc, étant entendu que le choix de cette technique sera fonction de la nature chimique de la surface du substrat, de la nature chimique et de la taille moléculaire du composé utilisé pour fonctionnaliser ladite zone sensible ainsi que du système de mesure du capteur de référence.

Parmi ces techniques, préférence est donnée, dans le cadre de l'invention, à l'auto-assemblage moléculaire.

Selon une autre disposition particulièrement préférée de l'invention, la surface du substrat est une surface passivée, c'est-à-dire qu'elle a été soumise à un traitement propre à minimiser les interactions physico-chimiques susceptibles de se produire entre cette surface et les composés de l'ensemble E et, partant, à réduire l'empoisonnement et le vieillissement des parties sensibles des capteurs de détection.

Auquel cas, la surface du substrat est, de préférence, passivée avec au moins un composé fluoré qui, lui également, est choisi parmi les composés comprenant au moins un groupe alkyle terminal perfluoré et les polymères fluorés et, en particulier, ceux précédemment mentionnés.

Conformément à l'invention, ce composé fluoré peut être le même composé que celui avec lequel la zone sensible correspondant à la partie sensible du capteur de référence est fonctionnalisée ou bien être un composé différent, pour autant qu'il soit choisi parmi les composés comprenant au moins un groupe alkyle terminal perfluoré et les polymères fluorés.

Comme précédemment indiqué, chacun des capteurs que comprend le système de détection peut comprendre son propre système de mesure - ou transducteur - ou partager avec d'autres capteurs un système de mesure qui leur est commun. Dans les deux cas, le système de mesure peut être tout système de mesure permettant de générer un signal exploitable lors de l'interaction physico-chimique entre un composé à l'état gazeux et la partie sensible d'un capteur et peut, notamment, être de type résistif, piézo-électrique, mécanique, acoustique ou optique. En d'autres termes, les capteurs peuvent être des capteurs résistifs, piézo-électriques, mécaniques, acoustiques et/ou optiques.

Toutefois, dans le cadre de l'invention, on préfère que les capteurs soient des capteurs optiques à résonance des plasmons de surface. Ce type de transduction, qui est connu en soi, combine généralement une source lumineuse, par exemple de type LED, afin de provoquer une excitation plasmonique et une caméra CCD pour enregistrer le signal résultant de la résonance plasmonique. À ce titre, on préfère tout particulièrement que les signaux émis par capteurs soient suivis en mode imagerie qui consiste à suivre les variations de signal de tous les pixels constituant l'image de la caméra CCD utilisée.

Le substrat est constitué d'un matériau adapté au système de mesure. Ainsi, si la mesure est réalisée par résonance des plasmons de surface, alors le substrat comprend, de préférence, un prisme en verre dont une face est recouverte d'une couche métallique, de préférence d'or ou d'argent, typiquement de 10 nm à 100 nm d'épaisseur. Cette couche métallique peut être passivée comme précédemment mentionné.

Comme il sera démontré dans les exemples qui suivent, l'invention présente de nombreux avantages.

En effet, en prévoyant de munir le système de détection d'un capteur de référence dont la fonction est de fournir un signal représentatif du bruit de mesure de ce système de détection, l'invention permet, outre de connaître ce bruit de mesure et, donc, de le soustraire des signaux émis par les capteurs de détection avec, à la clé, une plus grande fiabilité et une plus grande reproductibilité du fonctionnement du nez électronique, de détecter toute dérive du système de mesure ainsi que toute dérive des capteurs de détection et, partant, de corriger en conséquence, les signaux émis par les capteurs de détection avec, à la clé, là également, une plus grande fiabilité et une plus grande reproductibilité du fonctionnement du nez électronique.

En outre, en prévoyant de passiver la surface du substrat sur laquelle sont ou seront disposées les parties sensibles des capteurs de détection et du capteur de référence du système de détection, l'invention permet, de plus, de réduire la dérive des capteurs de détection, l'empoisonnement et le vieillissement des parties sensibles des capteurs de détection et, ainsi, de conférer ainsi une plus grande stabilité et une grande longévité au nez électronique.

L'invention a également pour objet un nez électronique apte à détecter et identifier un ensemble E de composés susceptibles d'être présents dans un échantillon gazeux, lequel nez électronique est caractérisé en ce qu'il comprend un système de détection tel que précédemment décrit.

Conformément à l'invention, le nez électronique est, de préférence, dédié à la détection et l'identification de composés organiques volatils, du sulfure d'hydrogène ($H_2S$) et de l'ammoniac ($NH_3$), ces composés pouvant se trouver seuls ou en mélange dans l'échantillon gazeux.

Dans ce qui précède et ce qui suit, un « composé organique volatil » est défini conformément à la Directive 1999/13/CE du Conseil Européen du 11 mars 1999 en vertu de laquelle :

- un composé organique volatil est « *tout composé organique ayant une pression de vapeur de 0,01 kPa (soit 9,87.10$^{-5}$ atm) ou plus à une température de 293,15 K (soit 20°C) ou ayant une volatilité correspondante dans les conditions d'utilisation particulières* » (cf. paragraphe 17 de l'article 2 de la Directive) ;
- un composé organique est « *tout composé comprenant au moins l'élément carbone et un ou plusieurs des éléments suivants : hydrogène, halogènes, oxygène, soufre, phosphore, silicium ou azote, à l'exception des oxydes de carbone et des carbonates et bicarbonates inorganiques* » (cf. paragraphe 16 de l'article 2 de la Directive).

Ainsi, sont notamment considérés comme des composés organiques volatils certains hydrocarbures acycliques, saturés ou insaturés, tels que l'éthane, le propane, le n-butane, le n-hexane, l'éthylène, le propylène, le 1,3-butadiène et l'acétylène, certains hydrocarbures cycliques non aromatiques, saturés ou insaturés, tels que le cyclopropane, le cyclopentane et le cyclohexane, certains hydrocarbures aromatiques tels que le benzène, le toluène, les xylènes et l'éthylbenzène, certains hydrocarbures halogénés tels que le dichlorométhane, le trichlorométhane, le chloroéthane, le trichloroéthylène et le tétrachloroéthylène, certains alcools tels que le méthanol, l'éthanol, le 1-propanol, le 2-propanol, l'éthylène glycol et le propylène glycol, certains aldéhydes tels que le formaldéhyde, l'acétaldéhyde, le propanal et le 2-propenal (ou acroléine), certaines cétones telles que l'acétone, la méthyléthylcétone, la 2-butanone et la méthylvinylcétone, certains esters tels que l'acétate de méthyle, l'acétate d'éthyle, l'acétate d'isopropyle et le butyrate d'isoamyle, certains éthers tels que l'éther diéthylique, le *n*-butyléther d'éthylène glycol (EGBE) et le 1,4-dioxane, certains acides tels que l'acide acétique et l'acide propanoïque, certaines amines telles que l'éthylamine, la diméthylamine, la triméthylamine, la diéthylamine et l'amylamine, certains amides tels que le diméthylformamide, les composés sulfurés tels que le mercaptan méthylique (ou méthanethiol) et le mercaptan éthylique (ou éthanethiol), et certains nitriles tels que l'acétonitrile et l'acrylonitrile.

D'autres caractéristiques et avantages de l'invention ressortiront du complément de description qui suit et qui est donné en référence aux figures annexées.

Il va de soi toutefois que ce complément de description n'est donné qu'à titre d'illustration de l'objet de l'invention et ne doit en aucun cas être interprété comme une limitation de cet objet.

## BRÈVE DESCRIPTION DES FIGURES

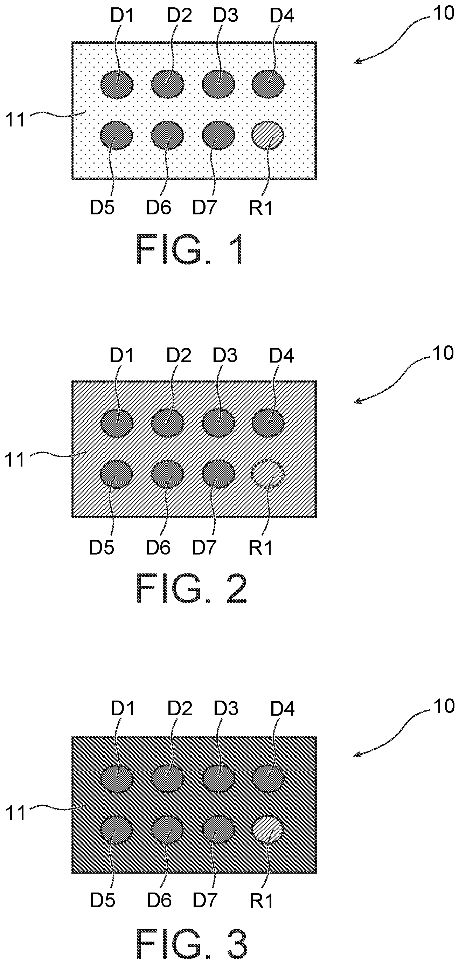

Les figures 1 à 3 illustrent schématiquement trois exemples de réalisation du substrat d'un système de détection selon l'invention, dans lequel les parties sensibles des capteurs de détection et des capteurs de référence sont agencées sur la surface d'un substrat commun.

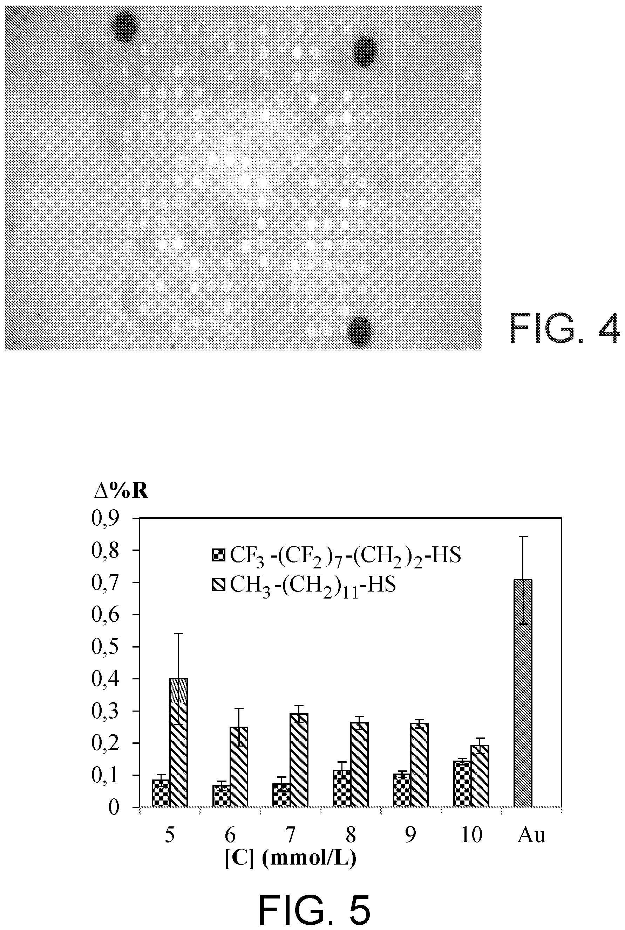

La figure 4 est une image différentielle obtenue par imagerie de résonance plasmonique de surface (ou SPRi) après exposition d'un système de détection selon l'invention, à substrat non passivé, à un échantillon gazeux comprenant un COV, en l'espèce le butyrate d'isoamyle.

La figure 5 illustre, sous la forme d'un diagramme en bâtons, la variation de la réflectivité, notée Δ%R, telle qu'obtenue par SPRi pour des couches auto-assemblées de 1*H*,1*H*,2*H*,2*H*-perfluorodécanethiol et de dodécanethiol agencées sur la surface d'un substrat, en fonction de la concentration, notée [C] et exprimée en mmol/L, des solutions à partir desquelles ces couches auto-assemblées ont été obtenues; est également représentée la variation de la réflectivité obtenue pour une zone de la couche d'or (bâton noté Au) formant la surface du substrat.

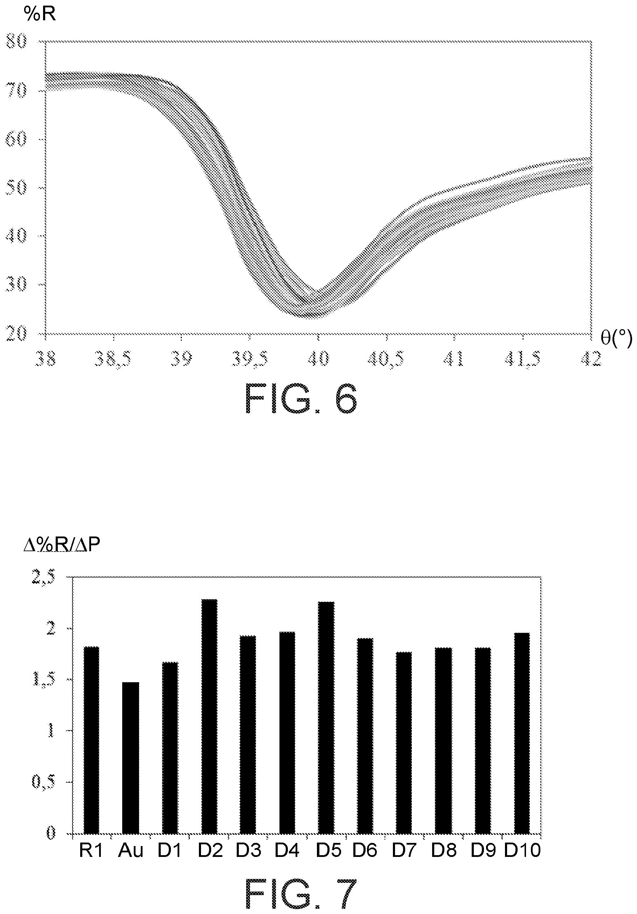

La figure 6 illustre les courbes des plasmons telles qu'obtenues par SPRi pour les capteurs de détection et les capteurs de référence d'un système de détection selon l'invention, à substrat non passivé, après exposition à un échantillon gazeux constitué uniquement d'air; sur cette figure, l'axe des ordonnées correspond à la réflectivité, notée %R, tandis que l'axe des abscisses correspond à l'angle d'incidence, noté θ et exprimé en degrés.

La figure 7 illustre, sous la forme d'un diagramme en bâtons, le rapport Δ%R/ΔP (variation de la réflectivité sur variation de pression) tel qu'obtenu par SPRi pour dix capteurs de détection, notés D1 à D10, et un capteur de référence, noté R1, d'un système de détection selon l'invention, à substrat non passivé, en réponse à une surpression (ΔP) ; est également représenté le rapport Δ%R/ΔP obtenu pour une zone de la couche d'or, notée Au, formant la surface du substrat.

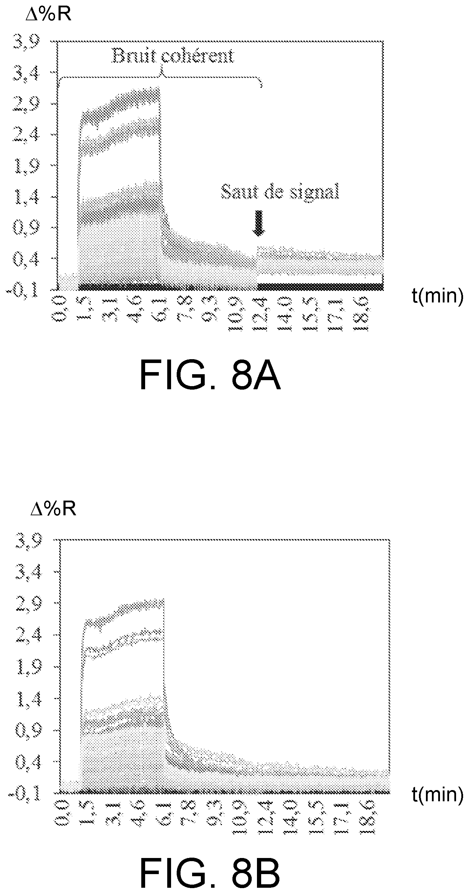

La figure 8A illustre la variation sans correction de la réflectivité, notée Δ%R, en fonction du temps, noté t et exprimé en minutes, telle qu'obtenue par SPRi pour une exposition à un échantillon gazeux comprenant du butyrate d'isoamyle, sur laquelle on observe un bruit cohérent et un saut de signal dus à des perturbations du système de mesure, tandis que la figure 8B correspond à la figure 8A mais après correction des données par le capteur de référence.

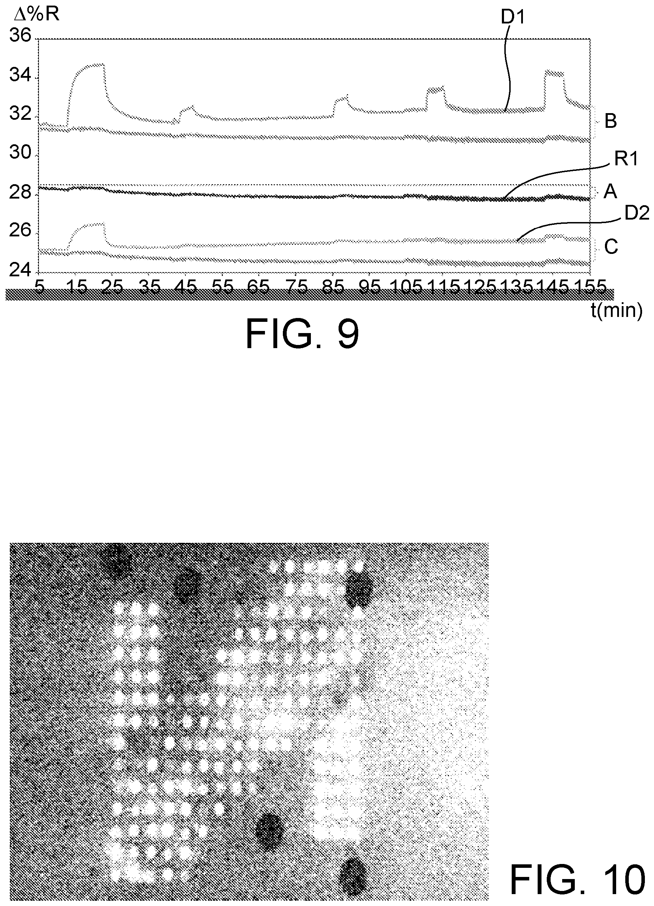

La figure 9 illustre l'évolution de la réflectivité, notée %R, en fonction du temps, noté t et exprimé en minutes, telle qu'obtenue par SPRi pour deux capteurs de détection (lignes D1 et D2) et un capteur de référence (ligne R1) d'un système de détection selon l'invention, à substrat non passivé, sur une période de 155 minutes au cours de laquelle ces capteurs ont été successivement exposés à cinq échantillons gazeux comprenant, pour le premier du butyrate d'isoamyle et pour les quatre autres, de l'amylamine ; sur cette figure, l'accolade A symbolise la dérive du système de mesure ; la ligne R1 a été dupliquée au-dessous des lignes D1 et D2 et les accolades B et C symbolisent la dérive chimique des capteurs D1 et D2 respectivement.

La figure 10 est une image différentielle obtenue par SPRi après exposition d'un système de détection selon l'invention, à substrat passivé, à un échantillon gazeux comprenant du butyrate d'isoamyle.

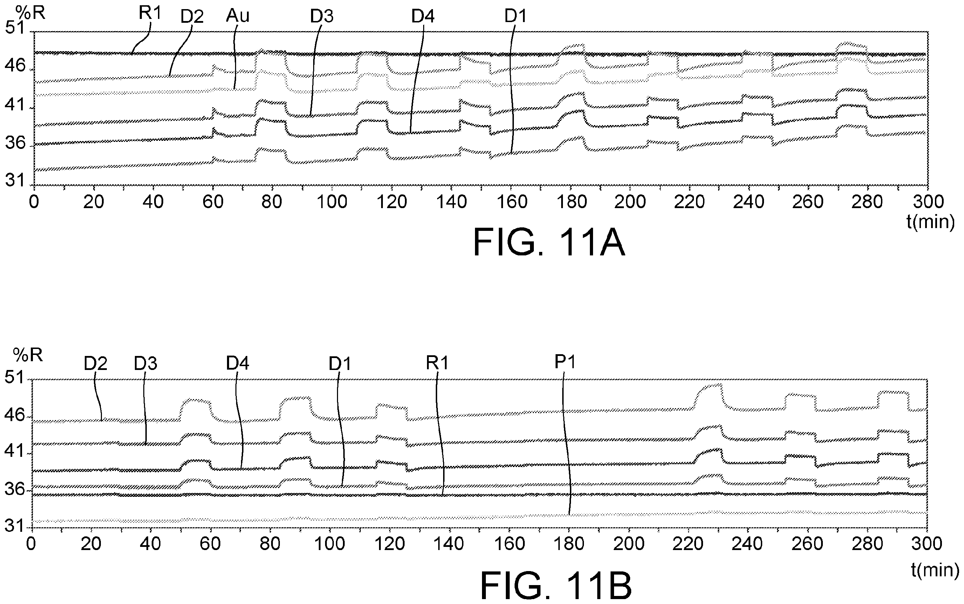

La figure 11A illustre l'évolution de la réflectivité, notée %R, en fonction du temps, noté t et exprimé en minutes, telle qu'obtenue par SPRi pour quatre capteurs de détection (lignes D1, D2, D3 et D4) et un capteur de référence (ligne R1) d'un système de détection selon l'invention, à substrat non passivé, sur une période de 300 minutes au cours de laquelle ces capteurs ont été successivement exposés à différents échantillons gazeux comprenant chacun un COV ; sur cette figure, est également montrée l'évolution de la réflectivité obtenue pour une zone de la couche d'or (ligne Au) formant la surface du substrat.

La figure 11B est une figure analogue à celle de la figure 11A mais pour un système de détection selon l'invention, à substrat passivé ; cette figure ne comprend donc pas de ligne Au mais comprend une ligne P1 correspondant à l'évolution de la réflectivité obtenue pour une zone de la surface du substrat passivé.

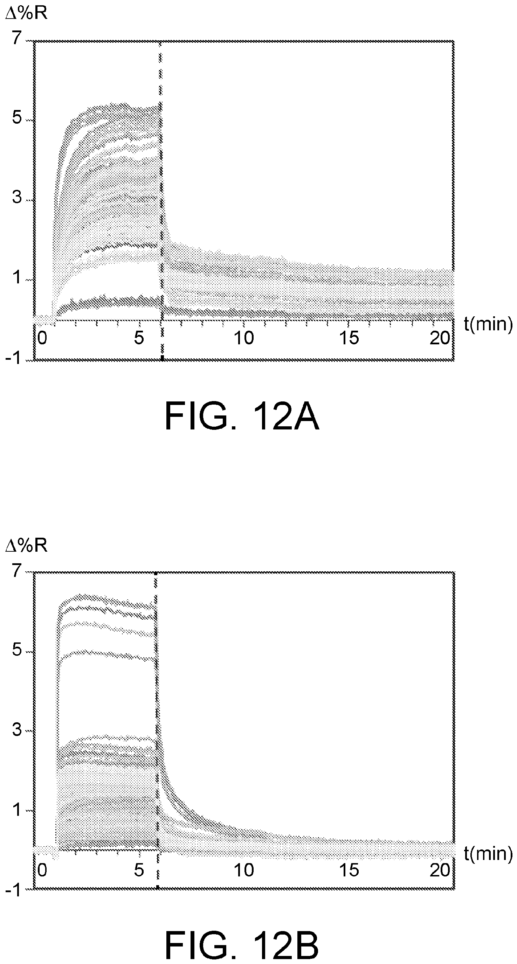

La figure 12A illustre la variation de la réflectivité, notée Δ%R, en fonction du temps, noté t et exprimé en minutes, telle qu'obtenue par SPRi pour des capteurs d'un système de détection selon l'invention, à substrat non passivé, au cours d'une exposition de 6 minutes à un échantillon gazeux comprenant de l'amylamine puis au cours d'un rinçage du substrat de 14 minutes sous flux d'air propre ; sur cette figure, la ligne verticale en pointillés symbolise la fin de l'exposition et le début du rinçage.

La figure 12B est une figure analogue à la figure 12A mais pour un système de détection selon l'invention, à substrat passivé.

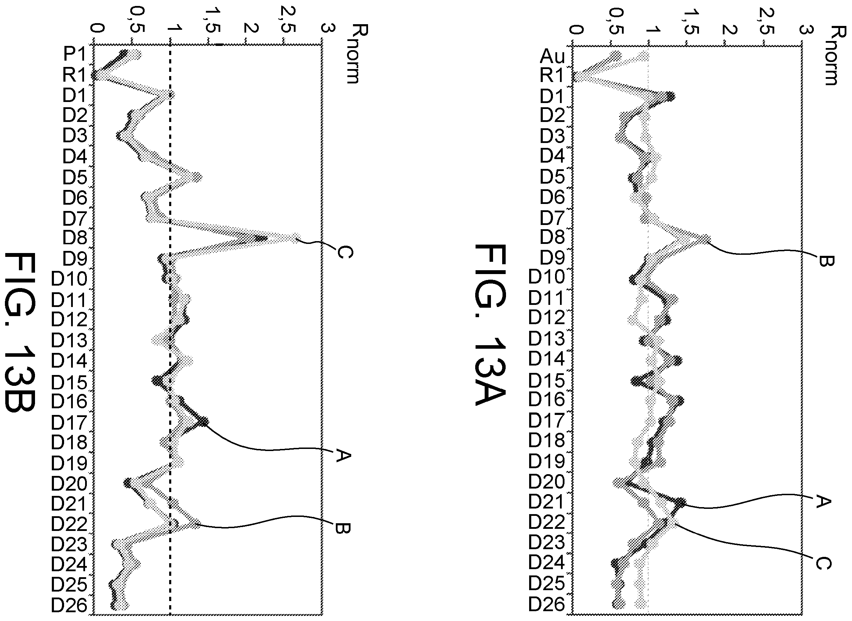

La figure 13A illustre la réflectivité normalisée, notée $R_{norm}$, telle qu'obtenue par SPRi pour vingt-six capteurs de détection, notés D1 à D26, et pour un capteur de référence, noté R1, d'un système de détection selon l'invention, à substrat non passivé, pour trois expositions à un échantillon gazeux comprenant du butyrate d'isoamyle ; entre chaque exposition au butyrate d'isoamyle, de l'amylamine a été injectée à plusieurs reprises ; sur cette figure, est également indiquée la réflectivité normalisée obtenue pour une zone de la couche d'or, notée Au, formant la surface du substrat; les points de la ligne A correspondent aux réflectivités normalisées obtenues avant le début des injections d'amylamine ; les points de la ligne B correspondent aux réflectivités normalisées obtenues au milieu des injections de l'amylamine tandis que les points de la courbe C correspondent aux réflectivités normalisées obtenues après la fin des injections d'amylamine.

La figure 13B est une figure analogue à la figure 13A mais pour un système de détection selon l'invention, à substrat passivé, et dans laquelle la réflectivité normalisée montrée pour la zone Au dans la figure 11A a donc été remplacée par celle obtenue pour une zone de la surface du substrat passivé, notée P1.

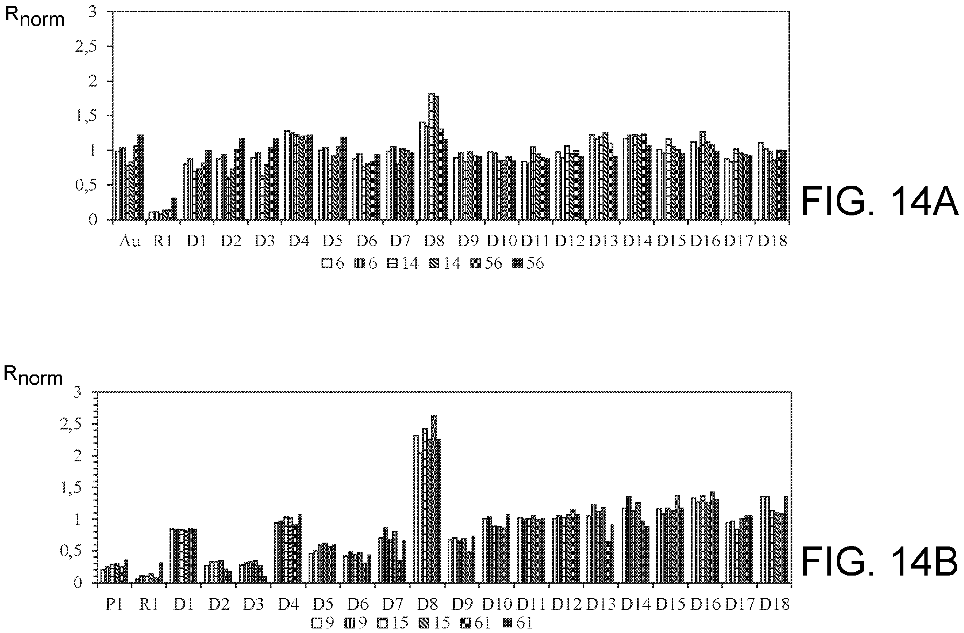

La figure 14A illustre la réflectivité normalisée, notée $R_{norm}$, telle qu'obtenue par SPRi pour dix-huit capteurs de détection, notés D1 à D18, et pour un capteur de référence, noté R1, d'un système de détection selon l'invention, à substrat non passivé, pour des expositions à un échantillon gazeux comprenant du butyrate d'isoamyle à 6, 14 et 56 jours d'utilisation ; sur cette figure, est également indiquée la réflectivité normalisée obtenue pour une zone de la couche d'or, notée Au, formant la surface du substrat.

La figure 14B est une figure analogue à la figure 14A mais pour un système de détection selon l'invention, à substrat passivé, ayant été exposé à un échantillon gazeux comprenant du butyrate d'isoamyle à 9, 15 et 61 jours d'utilisation ; sur cette figure, la réflectivité normalisée montrée pour la zone Au sur la figure 14A a donc été remplacée par celle obtenue pour une zone de la surface du substrat passivé, notée P1.

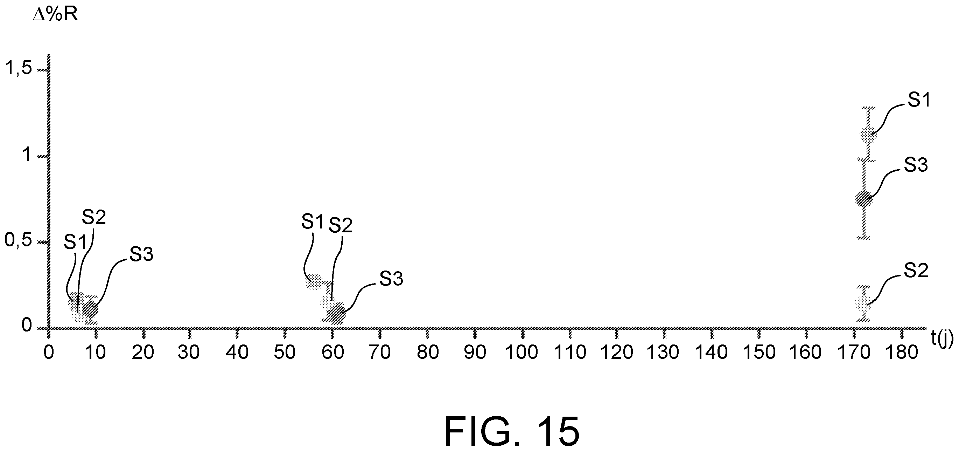

La figure 15 illustre la variation de réflectivité, notée $\Delta\%R$, en fonction du temps, noté t et exprimé en jours, telle qu'obtenue par SPRi pour un capteur de référence d'un système de détection selon l'invention, à substrat non passivé, noté S1, et pour un capteur de référence de deux systèmes de détection selon l'invention, à substrat passivé, notés S2 et S3, sur une période de 180 jours pendant laquelle ces capteurs ont été successivement exposés à trois échantillons gazeux comprenant du butyrate d'isoamyle.

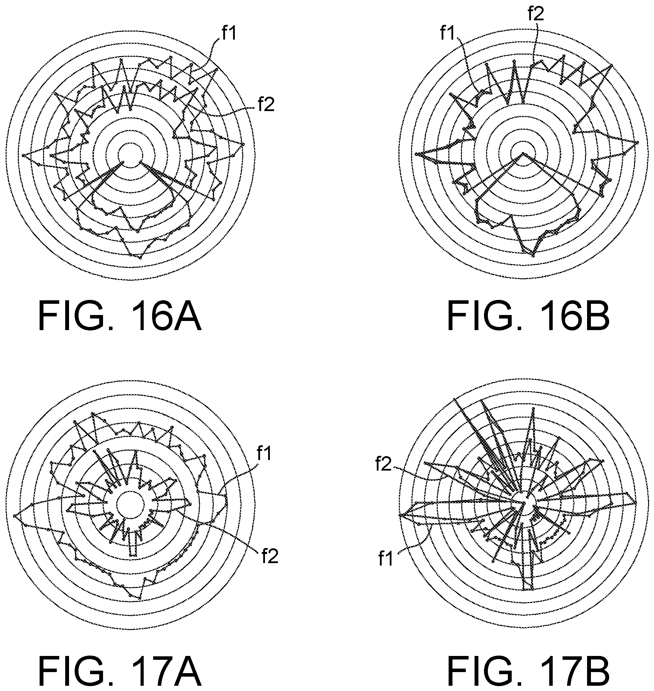

Les figures 16A et 16B illustrent, sous la forme de diagrammes en toile d'araignée, les signaux non normalisés (figure 16A) et normalisés (figure 16B) tels qu'émis par des capteurs d'un système de détection selon l'invention, à substrat non passivé, suite à deux expositions différentes : l'une à un échantillon de gaz de combustion d'essence, l'autre à un échantillon de vapeur d'eau ; sur ces figures, les flèches f1 correspondent aux signaux émis pour l'échantillon de gaz de combustion tandis que les flèches f2 correspondent aux signaux émis pour l'échantillon de vapeur d'eau.

Les figures 17A et 17B sont des figures analogues aux figures 16A et 16B mais pour un système de détection selon l'invention, à substrat passivé ; sur ces figures, les flèches f1 correspondent aux signaux émis pour l'échantillon de gaz de combustion tandis que les flèches f2 correspondent aux signaux émis pour l'échantillon de vapeur d'eau.

Sur les figures 1 à 3, les mêmes éléments sont désignés par les mêmes références.

## EXPOSÉ DÉTAILLÉ DE MODES DE RÉALISATION PARTICULIERS

## I - EXEMPLES DE RÉALISATION DU SUBSTRAT D'UN SYSTÈME DE DÉTECTION SELON L'INVENTION

On se réfère tout d'abord à la figure 1 qui illustre schématiquement un premier exemple de réalisation du substrat 10 d'un système de détection selon l'invention, dans lequel les parties sensibles des capteurs de détection et des capteurs de référence sont agencées sur la surface d'un même substrat.

Comme visible sur cette figure, le substrat 10 comprend une surface 11 sur laquelle est disposée une pluralité de zones sensibles référencées D1, D2, D3, D4, D5, D6 et D7 respectivement, qui correspondent chacune à la partie sensible d'un capteur de détection et qui seront donc dénommées « zones sensibles de détection » dans ce qui suit. Ces zones sensibles sont au nombre de 7 sur la figure 1 par souci de simplification de cette figure et correspondent donc à 7 capteurs de détection.

Toutefois, il va de soi qu'il est possible de réaliser un système de détection selon l'invention comprenant un nombre beaucoup plus élevé de capteurs de détection, par exemple 100, 500, 1 000, 3 000, voire 5 000 capteurs de détection, et, donc, un substrat sur la surface duquel est disposé un nombre correspondant de zones sensibles de détection.

La surface 11 du substrat 10 comprend également une zone sensible référencée R1, qui correspond à la partie sensible d'un capteur de référence et qui sera donc dénommée « zone sensible de référence » dans ce qui suit. Là également, par souci de simplification, la figure 1 ne comprend qu'une zone sensible de référence, correspondant à 1 capteur de référence.

Toutefois, là également, il est possible de réaliser un système de détection comprenant une pluralité de capteurs de référence, par exemple 5, 10, voire 50 capteurs de référence et, donc, un substrat sur la surface duquel est disposé un nombre correspondant de zones sensibles de référence.

Conformément à l'invention, les zones sensibles de détection D1 à D7 peuvent être créées en fonctionnalisant sept zones distinctes de la surface 11 du substrat 10 avec un ou plusieurs récepteurs, c'est-à-dire d'un ou plusieurs composés capables d'interagir de façon physico-chimique, typiquement par un mécanisme de sorption et, plus spécifiquement, d'adsorption, avec au moins l'un des composés de l'ensemble E de composés que le nez électronique est capable de détecter et d'identifier dans un échantillon gazeux.

Cette fonctionnalisation peut être réalisée au moyen de l'une quelconque des techniques de fonctionnalisation de surfaces connues de l'homme du métier (adsorption physique ou chimique, greffage covalent, épitaxie à jet moléculaire, dépôt de couches minces, auto-assemblage moléculaire, etc) ou par plusieurs d'entre elles, le choix de cette ou de ces techniques étant notamment fonction de la nature chimique de la surface du substrat, de la nature chimique et de la taille moléculaire des récepteurs ainsi que du système de mesure des capteurs de détection.

Dans le cas où les composés de l'ensemble E sont des COVs, $H_2S$ et $NH_3$, alors les récepteurs peuvent notamment être choisis parmi les métaux, les oxydes métalliques, les composés organiques non fluorés, les polymères non fluorés, les biomolécules comme des molécules d'ADN, des oligonucléotides, des sucres, des peptides, des protéines, des phospholipides, et les dérivés de ces biomolécules, ou encore parmi les différents arrangements moléculaires du carbone du type graphène, nanotubes, carbone graphite, etc. À cet égard, le lecteur pourra se référer aux articles de K. Arshak et al., Sensor Review 2004, vol. 24, pp. 181-198 ; T. Wasilewski et al., Biosensors and Bioelectronics 2017, vol. 87, pp. 480-494 ; et A. D. Wilson et M. Baietto, Sensors (Basel) 2009, vol. 9, pp. 5099-5148, ci-après références **[5]** à **[7],** pour de plus amples informations sur les types de récepteurs susceptibles d'être utilisés.

La zone sensible de référence R1 peut, elle, être créée en fonctionnalisant une zone de la surface 11 du substrat 10, qui est distincte des zones sensibles de détection D1 à D7, avec un ou plusieurs composés qui n'interagissent pas de façon physico-chimique avec les composés de l'ensemble E ou, s'ils interagissent avec l'un ou plusieurs de ces composés, donnent lieu à des interactions très faibles comparativement à celles qui sont susceptibles de se produire entre les récepteurs des zones sensibles D1 à D7 et lesdits composés de l'ensemble E.

Là également, cette fonctionnalisation peut être réalisée au moyen de l'une quelconque des techniques de fonctionnalisation de surfaces connues de l'homme du métier en fonction de la nature chimique du substrat, de la nature chimique et de la taille moléculaire du ou des composés ainsi que du système de mesure du capteur de référence.

Conformément à l'invention, le ou les composés n'interagissant pas ou que très faiblement avec les composés de l'ensemble E sont choisis parmi les composés fluorés comprenant au moins un groupe alkyle terminal perfluoré, c'est-à-dire au moins un groupe $-CF_3$, et les polymères fluorés en raison, d'une part, de l'inertie chimique qui caractérise ce type de composés et polymères et, d'autre part, de leurs propriétés non-mouillantes.

Des composés fluorés comprenant au moins un groupe alkyle terminal perfluoré peuvent notamment être des composés perfluorés, c'est-à-dire des composés de formule $C_vF_{2v+2}$ dans laquelle v est un entier allant de 4 à 20, ou des composés de formule $C_wF_{2w+1}-(L)_x-Z$ dans laquelle w est un entier allant de 1 à 12, x vaut 0 ou 1 et L représente un groupe divalent espaceur, par exemple un groupe hydrocarboné linéaire ou ramifié, saturé ou insaturé, comprenant de 1 à 20 atomes de carbone et éventuellement un ou plusieurs hétéroatomes, typiquement O, N, S et/ou Si, tandis que Z représente un groupe propre à permettre la fixation, par liaisons covalentes ou non, des composés sur la surface du substrat.

Pour une fonctionnalisation par auto-assemblage moléculaire - qui est la technique de fonctionnalisation qui est privilégiée dans le cadre de l'invention - le groupe Z peut, par exemple, être un groupe thiol (-SH) ou amine $(-NH_2)$ si la surface du substrat est en or, en platine, en argent, en palladium ou en cuivre, ou bien un groupe silanol (-SiOH) si la surface du substrat est en verre, en quartz, en silicium ou en silice.

Parmi les composés de formule $C_wF_{2w+1}-(L)_x-Z$, préférence est donnée aux perfluoralcanethiols de formule $CF_3(CF_2)_y(CH_2)_zSH$ dans laquelle y est un nombre entier allant de 0 à 11 et z est un nombre entier allant de 1 à 20 et, mieux encore, de 1 à 12, tels que le 1*H*,1*H*-trifluoroéthanethiol de formule $CF_3CH_2SH$, le 1*H*,1*H*,2*H*,2*H*-perfluoropentanethiol de formule $CF_3(CF_2)_2(CH_2)_2SH$, le 1*H*,1*H*,2*H*,2*H*-perfluorohexanethiol de formule $CF_3(CF_2)_3(CH_2)_2SH$, le 1*H*,1*H*,2*H*,2*H*-perfluorooctanethiol de formule $CF_3(CF_2)_5(CH_2)_2SH$ et le 1*H*,1*H*,2*H*,2*H*-perfluorodécanethiol de formule $CF_3(CF_2)_7(CH_2)_2SH$, tous disponibles auprès de la société SIGMA-ALDRICH.

Des polymères fluorés susceptibles d'être utilisés sont notamment les polytétrafluoroéthylènes (PTFE) comme ceux commercialisés par la société DUPONT sous la dénomination Teflon™, les fluorures de polyvinyle (PVF) comme ceux commercialisés par la société DUPONT sous la dénomination Tedlar™, les fluorures de polyvinylidène (PVDF) comme ceux commercialisés par la société ARKEMA sous la dénomination Kynar™, les perfluoroalcoxy alcanes (PFA) comme ceux commercialisés par la société DUPONT sous la dénomination Teflon™-PFA, les copolymères d'éthylène et de propylène fluorés (encore connus sous le nom d'éthylène-propylènes fluorés ou FEP) comme ceux commercialisés par la société DUPONT sous la dénomination Teflon™-FEP, et les poly(éthylène-co-tétrafluoroéthylène) (ETFE) comme ceux commercialisés par la société DUPONT sous la dénomination Tefzel™.

On se réfère à présent à la figure 2 qui illustre schématiquement un deuxième exemple de réalisation du substrat 10 d'un système de détection selon l'invention, dans lequel les parties sensibles des capteurs de détection et de référence sont également agencées sur un substrat commun.

Dans cet exemple, le substrat 10 ne diffère de celui représenté sur la figure 1 qu'en ce que sa surface 11 a été soumise à un traitement de passivation avec le même composé ou les mêmes composés que celui ou ceux formant la zone sensible de référence R1.

Dans le cas où les zones sensibles de détection D1 à D7 sont créées par greffage covalent ou par auto-assemblage moléculaire et où le traitement de passivation est lui-même effectué au moyen de l'une de ces deux techniques, alors ce traitement de passivation peut être réalisé après la création des zones sensibles de détection D1 à D7, auquel cas sont passivées uniquement les parties de la surface 11 du substrat 10 laissées libres par les zones sensibles de détection D1 à D7 et la zone sensible de référence R1 est choisie sur l'une de ces parties passivées.

En variante, le traitement de passivation peut aussi être réalisé avant la création des zones sensibles de détection D1 à D7, auquel cas ces zones sensibles peuvent être créées soit en recouvrant, au niveau de sept zones distinctes de la surface 11 du substrat 10, le ou les composés de passivation présents sur ces zones d'un ou plusieurs récepteurs soit en dépassivant sept zones distinctes de la surface 11 du substrat 10, par exemple par lithographie, et en fonctionnalisant les zones ainsi dépassivées avec un ou plusieurs récepteurs. Là également, la zone sensible de référence R1 peut ensuite être choisie sur l'une des parties de la surface 11 du substrat 10 laissées libres par les zones sensibles de détection D1 à D7.

La figure 3 illustre schématiquement une variante du deuxième exemple de réalisation du substrat 10 d'un système de détection selon l'invention, variante dans laquelle la surface 11 du substrat 10 a été soumise à un traitement de passivation avec un ou des composés qui sont différents de celui ou ceux formant la zone sensible de référence R1 mais qui sont également choisis parmi les composés comprenant un groupe alkyle terminal perfluoré et les polymères fluorés.

Ainsi, par exemple, il est possible d'utiliser deux composés différents choisis parmi les composés perfluorés et les composés de formule $C_wF_{2w+1}$-$(L)_x$-$Z$ telle que précédemment définie pour former la zone sensible de référence R1 et passiver la surface 11 du substrat.

En variante, il est également possible d'utiliser un composé perfluoré ou un composé de formule $C_wF_{2w+1}$-$(L)_x$-$Z$ telle que précédemment définie pour former la zone sensible de référence R1 et de passiver la surface 11 du substrat 10 avec un polymère fluoré tel que le PTFE.

Là également, le traitement de passivation peut être réalisé après ou avant la création des zones sensibles de détection D1 à D7 et de la zone sensible de référence R1 selon les mêmes modalités que celles décrites ci-dessus.

Il est à noter que les figures 1 à 3 correspondant à des illustrations schématiques de modes de réalisation du système de détection selon l'invention, les zones sensibles D1 à D7 et R1 sont représentées circulaires sur ces figures.

Toutefois, il va de soi que les zones sensibles de détection et les zones sensibles de référence peuvent avoir une toute autre forme, par exemple polygonale et, en particulier parallélépipédique, ou être de forme quelconque.

De manière similaire, les zones sensibles D1 à D7 et R1 sont représentées séparées les unes des autres sur les figures 1 à 3 mais il est tout à fait envisageable que les zones sensibles de détection et les zones sensibles de référence soient accolées les unes aux autres.

## II - PROPRIÉTÉS D'UN SYSTÈME DE DÉTECTION SELON L'INVENTION À SUBSTRAT NON PASSIVÉ

### II.1 - Mise en évidence par imagerie de la différence de sensibilité existant entre les capteurs de détection et les capteurs de référence vis-à-vis des COVs :

Le présent test a été réalisé en utilisant un substrat constitué d'un prisme en verre, recouvert sur l'une de ses faces d'une couche de chrome (d'environ 2 nm d'épaisseur), elle-même recouverte d'une couche d'or (d'environ 50 nm d'épaisseur) et comprenant :

- une pluralité de zones sensibles de détection, ces zones étant formées de couches auto-assemblées de différents récepteurs à réactivité croisée (c'est-à-dire qu'un récepteur peut interagir avec différents COVs et, à l'inverse, un COV peut réagir avec différents récepteurs), chacun des récepteurs comprenant une fonction thiol pour sa fixation sur la couche d'or, et
- une pluralité de zones sensibles de référence, ces zones étant formées de couches auto-assemblées de 1*H*,1*H*,2*H*,2*H*-perfluorodécanethiol, cet auto-assemblage ayant été réalisé par microdépôt, au moyen d'un robot, d'une solution comprenant 7 mmol/L de ce composé sur la couche d'or.

Ce substrat a été exposé pendant 10 minutes à un échantillon gazeux comprenant un COV, en l'espèce le butyrate d'isoamyle $(CH_3(CH_2)_2C(O)O(CH_2)_2CH(CH_3)_2)$ à une concentration d'environ 30 ppm, et les interactions entre ce COV et les différentes zones sensibles du substrat ont été suivies par imagerie de résonance des plasmons de surface (ou SPRi).

A ainsi été obtenue l'image différentielle montrée sur la figure 4.

La SPRi est une technique de lecture optique des interactions de surface qui peut être assimilée à une balance optique : plus il y a d'interactions entre un composé et une surface, plus le signal optique émis par le système de lecture augmente. Une image différentielle représente la variation de ce signal optique par rapport à une image de référence prise avant l'exposition de la surface au composé. Aussi, plus une zone de l'image différentielle est claire, plus l'interaction physico-chimique entre le composé et la zone correspondante de la surface est élevée. À l'inverse, plus une zone de l'image différentielle est sombre, plus l'interaction physico-chimique entre le composé et la zone de la surface est faible.

Comme le montre la figure, l'image différentielle présente des zones qui sont très claires, voire blanches, et qui correspondent aux zones sensibles de détection du substrat sur lesquelles le butyrate d'isoamyle s'est adsorbé et des zones qui sont noires et qui correspondent aux zones sensibles de référence du substrat sur lesquelles le butyrate d'isoamyle ne s'est pas ou que très peu adsorbé, ce qui démontre que des zones d'un substrat qui sont fonctionnalisées par le 1*H*,1*H*,2*H*,2*H*-perfluorodécanethiol ont une réelle inertie chimique vis-à-vis des COVs comme le butyrate d'isoamyle.

## II.2 - Comparaison entre la sensibilité vis-à-vis des COVs de capteurs dont les parties sensibles sont formées d'un perfluoroalcanethiol et celle de capteurs dont les parties sensibles sont formées d'un alcanethiol non fluoré :

Le présent test a été réalisé en utilisant une série de substrats constitués de prismes en verre, recouverts sur l'une de leurs faces d'une couche d'or (d'environ 50 nm d'épaisseur) et comprenant, d'une part, des zones formées de couches auto-assemblées de 1*H*,1*H*,2*H*,2*H*-perfluorodécanethiol et, d'autre part, des zones formées de couches auto-assemblées de dodécanethiol $(CH_3(CH_2)_{11}SH)$, les auto-assemblages ayant été obtenus par micro-dépôt, au moyen d'un robot, de solutions comprenant de 5 mmol/L à 10 mmol/L de l'un de ces composés sur la couche d'or.

Ces substrats ont été exposés à des échantillons gazeux comprenant 20 ppm de butyrate d'isoamyle et les interactions entre ce COV et les différentes couches auto-assemblées présentes sur les substrats ont été suivies par SPRi.

Les résultats de ce test sont illustrés sur la figure 5 qui montre, sous la forme d'un diagramme en bâtons, la variation de la réflectivité, notée Δ%R, telle qu'obtenue pour chacun des deux types de couches et pour chacune des concentrations, notées [C] et exprimées en mmol/L, des solutions à partir desquelles ces couches ont été obtenues. À titre de comparaison, la figure 5 montre également la variation de la réflectivité obtenue pour une zone de la couche d'or (bâton noté Au).

Comme le montre cette figure, la variation de la réflectivité observée pour les couches auto-assemblées de dodécanethiol - qui traduit la sensibilité de ces couches vis-à-vis du butyrate d'isoamyle -, bien que plus faible que celle observée pour la couche d'or, est systématiquement plus élevée que celle observée pour les couches de 1*H*,1*H*,2*H*,2*H*-perfluorodécanethiol, et ce, quelle que soit la concentration de la solution utilisée pour le dépôt de ces couches.

Il en résulte que l'adsorption du butyrate d'isoamyle sur les couches de dodécanethiol est systématiquement plus élevée que ce qu'elle est sur les couches de 1*H*,1*H*,2*H*,2*H*-perfluorodécanethiol.

Comme le montre également cette figure, la variation de la réflectivité des couches auto-assemblées dépend de la concentration de la solution utilisée pour le dépôt de ces couches. Dans le cas du 1*H*,1*H*,2*H*,2*H*-perfluorodécanethiol, la variation de la réflectivité la plus faible est observée pour une concentration allant de 6 mmol/L à 7 mmol/L, concentration à laquelle cette variation est plus de deux fois plus faible que celle obtenue pour les couches auto-assemblées de dodécanethiol.

## II.3 - Vérification de la fonctionnalité des capteurs de référence :

Pour s'assurer que les zones sombres observées sur l'image de la figure 4 sont bien dues à une absence ou quasi absence d'interaction physico-chimique entre le butyrate d'isoamyle et les zones sensibles de référence du substrat décrit dans l'exemple II.1 ci-avant, ce substrat a été exposé à un échantillon gazeux constitué uniquement d'air (et, donc, exempt de tout composé chimique susceptible d'interagir avec zones sensibles de détection) et les courbes des plasmons ont été établies par SPRi pour les zones sensibles de détection et de référence.

Ces courbes sont illustrées sur la figure 6.

Comme le montre cette figure, les courbes des plasmons obtenues pour les zones sensibles de référence se confondent avec celles obtenues pour les zones sensibles de détection.

Les zones sombres observées sur l'image de la figure 4 sont donc bien due à une absence ou quasi absence d'interaction physico-chimique entre le butyrate d'isoamyle et les zones sensibles de référence du substrat et non au fait que la SPRi serait hors de son domaine de sensibilité.

**II.4** - **Sensibilité des capteurs de référence aux variations de paramètres physiques** :

Afin de vérifier que les zones sensibles de référence du substrat décrit dans l'exemple II.1 ci-avant, bien qu'insensibles ou que très peu sensibles à la présence de COVs, sont néanmoins sensibles à des variations de paramètres physiques environnementaux ou expérimentaux, un test dit de « saut d'indice » a été effectué.

Le principe de ce test est de faire varier l'indice de réfraction d'un échantillon gazeux constitué uniquement d'air (et, donc, exempt de tout composé chimique susceptible d'interagir chimiquement avec les zones sensibles de détection du substrat) au moyen d'un paramètre physique et d'observer si la variation de l'indice de réfraction se traduit ou non par une variation des signaux optiques obtenus par SPRi pour les zones sensibles de détection et les zones sensibles de référence du substrat.

Dans le cas présent, le test a été réalisé en appliquant une surpression ($\Delta P$ = 0,5 bar) au substrat.

Les résultats de ce test sont présentés sur la figure 7, sous la forme d'un diagramme en bâtons exprimant le rapport $\Delta\%R/\Delta P$ obtenu pour une zone sensible de référence, notée R1, dix zones sensibles de détection, notées D1 à D10, et pour une zone de la couche d'or, notée Au, du substrat.

Comme le montre cette figure, une variation de l'indice de réfraction de l'échantillon gazeux ayant pour seule origine une variation d'un paramètre physique s'est traduite par un rapport $\Delta\%R/\Delta P$ qui est comparable pour toutes les zones sensibles du substrat.

La sensibilité à des variations de paramètres physiques peut donc être considérée comme étant la même pour toutes les zones sensibles du substrat.

Les zones sensibles de référence ne sont donc pas ou que très peu sensibles aux COVs tout en étant sensibles à des variations de paramètres physiques, ici une variation de pression. Elles peuvent donc bien être utilisées comme références négatives ou références zéro des interactions physico-chimiques susceptibles de se produire sur le substrat avec des COVs.

**II.5 - Évaluation du bruit de mesure :**

Dans la mesure où, comme montré dans l'exemple précédent, les zones sensibles de référence du substrat décrit dans l'exemple II.1 ci-avant sont sensibles à des variations de paramètres physiques, le bruit de mesure d'un système de détection comprenant un tel substrat peut être estimé en mesurant la fluctuation à court terme des signaux optiques obtenus pour ces zones sensibles lors d'une exposition à un échantillon gazeux comprenant un COV tel que le butyrate d'isoamyle.

La figure 8A montre que le bruit de mesure normalement mesuré est d'environ $\pm$ 0,05 %R (cf. de 14 à 18 minutes).

Sur cette figure, on observe également un bruit cohérent, c'est-à-dire un bruit qui se répercute de la même manière sur l'ensemble des signaux optiques émis par les capteurs (cf. de 0 à 12 minutes), ainsi qu'un saut de signal. Ces phénomènes sont dus à des paramètres extérieurs, par exemple des vibrations pour le bruit cohérent ou une variation brusque de pression pour le saut de signal. Comme ils ne sont pas reliés à la détection chimique, ils peuvent être éliminés grâce aux capteurs de référence d'un système de détection selon l'invention, en soustrayant le signal optique moyen obtenu à un temps t pour les zones sensibles de référence à celui obtenu au même temps t pour chacune des zones sensibles de détection.

On obtient ainsi, pour chaque zone sensible de détection, un signal optique « nettoyé » comme visible sur la figure 8B.

**II.6** - **Évaluation de la dérive du système de mesure et de la dérive de chaque capteur de détection :**

Un test consistant à exposer le substrat décrit dans l'exemple II.1 ci-avant successivement à cinq échantillons gazeux comprenant, pour le premier 22 ppm de butyrate d'isoamyle, pour le deuxième 3,2 ppm d'amylamine, pour le troisième 4,2 ppm d'amylamine, pour le quatrième 5 ppm d'amylamine et pour le cinquième 14,20 ppm d'amylamine, sur une durée totale de 155 minutes et à suivre par SPRi l'évolution de la réflectivité pour une zone sensible de référence, notée R1, et pour deux zones sensibles de détection de ce substrat, notées D1 et D2, pendant toute la durée de cette exposition.

A ainsi été établie la figure 9 dans laquelle la ligne notée R1 correspond à l'évolution de la réflectivité, noté %R, telle qu'observée pour la zone R1, tandis que les lignes notées D1 et D2 représentent l'évolution de %R telle qu'observée pour les zones D1 et D2 respectivement. Sur cette figure, la ligne R1, qui correspond à la dérive du système de mesure, a été dupliquée en dessous de chacune des lignes D1 et D2.

Comme précédemment mentionné, la dérive du système de mesure est une variation progressive dans le temps du niveau moyen du bruit de mesure, ou ligne de base du signal, qui peut être causée par des phénomènes complexes, pouvant notamment être liés aux variations environnementales (par exemple, un gradient de température dans le cas de la SPRi), à des contaminations croisées, à la pollution du nez électronique par des composés dits « poisons », etc.

Comme les capteurs de référence ne sont sensibles qu'à des variations de paramètres physiques, la dérive du système de mesure peut être évaluée comme la dérive du signal émis par ces capteurs de référence comme illustré par l'accolade notée A sur la figure 9. Cette dérive affectant aussi les capteurs de détection, elle peut être corrigée en soustrayant le signal émis par un capteur de référence aux signaux émis par les capteurs de détection.

Comme également précédemment mentionné, la dérive d'un capteur correspond à la somme de la dérive du système de mesure et de la dérive chimique de capteur, causée par exemple par un empoisonnement ou une pollution chimique progressive de la partie sensible du capteur.

En suivant uniquement le signal émis par un capteur de détection donné, il est impossible de distinguer ces deux sources de dérive. Par contre, la différence entre le signal émis par un capteur de détection et celui émis par un capteur de référence permet de quantifier la dérive chimique, capteur par capteur, comme illustré par les accolades notées B et C sur la figure 9.

Pouvoir déterminer la dérive chimique seule de chaque capteur de détection permet de tester l'efficacité de solutions expérimentales pour la diminuer au maximum. Ainsi, le nez électronique peut être rendu plus fiable et reproductible à long terme.

### III - PROPRIÉTÉS D'UN SYSTÈME DE DÉTECTION SELON L'INVENTION À SUBSTRAT PASSIVÉ

### III.1 - Mise en évidence par imagerie de l'effet d'une passivation sur l'adsorption de COVs sur un substrat :

Le présent test a été réalisé en passivant la surface d'un substrat tel que décrit dans l'exemple II.1 ci-avant mais présentant un motif de fonctionnalisation différent de celui-ci (c'est-à-dire une répartition différente des zones sensibles de détection et de référence), au moyen du trifluoroéthanethiol qui, comme le $1H,1H,2H,2H$-perfluorodécanethiol, est un composé à groupe alkyle perfluoré mais dont la chaîne carbonée est plus courte (2 atomes de carbone au lieu de 10).

Cette passivation a été réalisée par diffusion sur la surface du substrat du trifluoroéthanethiol en phase liquide.

Ce substrat a été exposé à un échantillon gazeux comprenant du butyrate d'isoamyle dans les mêmes conditions que celles décrites dans l'exemple II.1 ci-avant et les interactions entre ce COV et les différentes zones sensibles du substrat ont été suivies, là également, par SPRi.

A ainsi été obtenue l'image différentielle montrée sur la figure 10, dans les mêmes conditions de contraste et de luminosité que celles utilisées pour l'image de la figure 4.

Une comparaison de ces deux images montre que la partie de la surface du substrat laissée libre par les zones sensibles de détection et de référence apparaît plus sombre sur l'image de la figure 10, ce qui signifie que l'adsorption du butyrate d'isoamyle sur cette surface est réduite par rapport à ce qu'elle est sur la surface d'un substrat non passivé.

### III.2 - Diminution de la dérive chimique des capteurs :

Un test a été réalisé en exposant successivement :

- d'une part, un substrat non passivé tel que décrit dans l'exemple II.1 ci-avant, à sept échantillons gazeux comprenant, pour les deux premiers du butyrate d'isoamyle (44,2 ppm et 37,1 ppm), pour le troisième de l'éthanol (173 ppm), pour le quatrième du 1-octanol (4,2 ppm), pour le cinquième du 1-propanol (192 ppm), pour le sixième du 1-butanol (75,6 ppm) et pour le septième du butyrate d'isoamyle (40,6 ppm), et

- d'autre part, un substrat passivé tel que préparé dans l'exemple III.1 ci-avant, à six échantillons gazeux comprenant, pour les deux premiers du butyrate d'isoamyle (47,9 ppm et 53,2 ppm), pour le troisième de l'éthanol (168 ppm), pour le quatrième du 1-octanol (3,8 ppm), pour le cinquième du 1-propanol (165 ppm) et pour le sixième du 1-butanol (82,1 ppm),

et ce, sur une période de 300 minutes et en suivant par SPRi et pour chaque substrat, l'évolution de la réflectivité, %R, pour une zone sensible de référence, notée R1, et pour quatre zones sensibles de détection, notées D1 à D4, sur cette période.

A également été suivie l'évolution de la réflectivité pour une zone de la couche d'or, notée Au, du substrat non passivé et pour une zone de la surface du substrat passivé, notée P1.

Ont ainsi été établies les figures 11A et 11B, la figure 11A correspondant au substrat non passivé et la figure 11B correspondant au substrat passivé.

Une comparaison de ces figures montre que la passivation permet de diminuer la dérive chimique pour l'ensemble des zones sensibles de détection D1 à D4. Sur 300 minutes (soit 5 heures) d'analyse, la dérive chimique peut ainsi être divisée par deux, passant de 0,6 %R.h$^{-1}$ (substrat non passivé) à 0,3 %R.h$^{-1}$ (substrat passivé). Pour l'analyse d'un COV sur dix minutes, la dérive chimique obtenue devient de l'ordre du bruit de mesure, soit 0,05 %R.

### III.3 - Résistance du système de détection à un « effet poison » :

Certains COVs ont une affinité particulière pour les substrats recouverts d'une couche d'or et leur fixation non réversible sur ce type de substrats peut se traduire par un empoisonnement du système de détection avec à la clé, une baisse de fiabilité et de reproductibilité de la détection assurée par ce système. Les amines et les thiols s'adsorbent, par exemple, de manière irréversible sur l'or. Ces composés peuvent cependant être des cibles d'intérêt pour certaines applications, notamment pour l'agro-alimentaire dans le cas des amines.

Deux tests ont donc été réalisés pour vérifier si la passivation d'un substrat recouvert d'une couche d'or pouvait réduire l'empoisonnement de ce substrat.

Le premier test a été réalisé en exposant, d'une part, un substrat non passivé tel que décrit dans l'exemple II.1 ci-avant, et, d'autre part, un substrat passivé tel que préparé dans l'exemple III.1 ci-avant, à un échantillon gazeux comprenant une amine, en l'espèce l'amylamine ($CH_3(CH_2)_4NH_2$), pendant cinq minutes aux termes desquelles les substrats ont été rincés pendant 14 minutes sous un flux d'air propre.

Le deuxième test a été réalisé en exposant ces mêmes substrats à une série de trois expositions à un échantillon gazeux comprenant du butyrate d'isoamyle. Entre chaque exposition, de l'amylamine a été injectée à plusieurs reprises à une concentration allant de 3 ppm à 50 ppm.

Dans les deux tests, l'évolution de la réflectivité, %R, a été suivie par SPRi pour une zone sensible de référence, notée R1, et pour vingt-six zones sensibles de détection, notées D1 à D26. A également été suivie l'évolution de la réflectivité pour une zone de la couche d'or, notée Au, du substrat non passivé et pour une zone de la surface du substrat passivé, notée P1.

Les résultats du premier test sont illustrés, exprimés en variations de réflectivité (Δ%R), sur les figures 12A et 12B, tandis que les résultats du deuxième test sont illustrés, exprimés en réflectivités normalisées ($R_{norm}$), sur les figures 13A et 13B.

$$R_{norm} = \frac{\Delta\%R_D}{\sum_n \Delta\%R_{Dn}}.$$

$N_D$ où $\Delta\%R_D$ est la variation de réflectivité pour un capteur de détection D et $N_D$ le nombre total de capteurs de détection.

Ces figures montrent que, dans le cas du substrat non passivé (Figures 12A et 13A), les signaux émis par la majorité des capteurs ne reviennent pas à la ligne de base après rinçage du substrat sous flux d'air propre. L'amylamine s'est donc fixée de manière irréversible dans les conditions d'expérimentation.

Par contre, dans le cas du substrat passivé (figures 12B et 13B), le retour à la ligne de base est bien meilleur et donc la fixation irréversible de l'amylamine a été limitée.

Les figures 13A et 13B mettent de plus en évidence une amélioration de la répétabilité du système de détection avec la passivation. En effet, le profil obtenu pour le butyrate d'isoamyle est conservé même après exposition à un composé poison tel que l'amylamine.

### III.4 - Amélioration du vieillissement du système de détection :

Deux tests ont donc été réalisés pour vérifier si la passivation d'un substrat recouvert d'une couche d'or pouvait réduire le vieillissement du système de détection.

Le premier test a réalisé en exposant, d'une part, un substrat non passivé tel que décrit dans l'exemple II.1 ci-avant, et, d'autre part, un substrat passivé tel que préparé dans l'exemple III.1 ci-avant, à des échantillons gazeux comprenant de 35 ppm à 55 ppm de butyrate d'isoamyle, au 6ème jour (avec 3 expositions), au 14ème jour (avec 2 expositions) et au 56ème jour (avec 2 expositions) d'utilisation pour le premier substrat et au 9ème jour (avec 3 expositions), au 15ème jour (avec 2 expositions) et au 61ème jour (avec 2 expositions) d'utilisation pour le second substrat, et en suivant par SPRi et pour chacun substrats l'évolution de la réflectivité pour une zone sensible de référence, notée R1, et pour dix-huit zones sensibles de détection, notées D1 à D18. A également été suivie l'évolution de la réflectivité pour une zone de la couche d'or, notée Au, du substrat non passivé et pour une zone de la surface du substrat passivé, notée P1.

Le deuxième test a été réalisé en exposant :

- un substrat non passivé tel que décrit dans l'exemple II.1 ci-avant, dénommé ci-après S1,
- un substrat passivé tel que préparé dans l'exemple III.1 ci-avant, c'est-à-dire passivé par diffusion liquide de trifluoroéthanethiol, dénommé ci-après S2, et
- un substrat préparé en passivant la surface d'un substrat tel que décrit dans l'exemple II.1 ci-avant, par diffusion liquide de 1*H*,1*H*,2*H*,2*H*-perfluorodécanethiol, dénommé ci-après S3,

à des échantillons gazeux comprenant de 35 ppm à 55 ppm de butyrate d'isoamyle, à 1 semaine, à 2 mois et à 6 mois d'utilisation de ces substrats, et en suivant par SPRi et pour chacun des substrats S1, S2 et S3, l'évolution de la réflectivité pour une zone sensible de référence.

Les résultats du premier test sont illustrés, exprimés en réflectivités normalisées ($R_{norm}$), sur les figures 14A et 14B tandis que les résultats du deuxième test sont illustrés, exprimés en variations de réflectivité (Δ%R), sur la figure 15.

Les figures 14A et 14B montrent que, dans le cas d'un substrat non passivé (S1), le profil obtenu sur l'ensemble des capteurs se détériore dans le temps car la réponse de chaque capteur tend vers la même valeur. Ainsi, on perd la capacité du nez électronique à différencier des COVs. En revanche, dans le cas d'un substrat passivé (S2 et S3), le profil obtenu sur l'ensemble des capteurs est bien conservé.

La figure 15 montre, elle, que l'insensibilité ou la très faible sensibilité aux COVs des capteurs de référence est également mieux conservée avec une passivation du substrat. En utilisant le trifluoroéthanethiol comme composé passivant (S2), les capteurs de référence sont même stabilisés jusqu'à six mois d'utilisation.

**III.5** - **Réduction des interférences avec l'eau présente dans un échantillon gazeux** :

Le présent test a été réalisé en utilisant deux substrats tels que décrits dans l'exemple II.1 ci-avant mais présentant un motif de fonctionnalisation différent de celui-ci (c'est-à-dire une répartition différente des zones sensibles de détection et de référence).

Le premier substrat n'a pas été passivé tandis que le deuxième substrat a été passivé au moyen de 1*H*,1*H*,2*H*,2*H*-perfluorohexanethiol.

Cette passivation a été réalisée par diffusion sur la surface du deuxième substrat du 1*H*,1*H*,2*H*,2*H*-perfluorohexanethiol en phase liquide à la concentration de 1 mmol/L dans l'éthanol.

Chaque substrat a été exposé à un échantillon gazeux comprenant des gaz de combustion d'essence, provenant d'un échappement d'un véhicule à essence.

Les interactions entre ces gaz de combustion et les différentes zones sensibles des deux substrats ont été suivies par SPRi.

Les produits de combustion comprenant beaucoup de vapeur d'eau (environ 70% d'humidité relative) du fait que l'eau est un produit de combustion de l'essence, ces interactions ont été comparées avec celles obtenues pour de la vapeur d'eau seule.

Les résultats sont illustrés, sous la forme de diagrammes en toile d'araignée (ou diagramme en radar), sur les figures 16A et 16B pour le substrat non passivé et sur les figures 17A et 17B pour le substrat passivé. Dans ces diagrammes, chaque rayon correspond au signal émis par une zone sensible du substrat, en intensité absolue pour les figures 16A et 17A et en intensité normalisée pour les figures 16B et 17B. Les flèches f1 correspondent aux signaux émis pour les échantillons de gaz de combustion d'essence tandis que les flèches f2 correspondent aux signaux émis pour les échantillons de vapeur d'eau.

Les figures 16A et 16B montrent que, pour un substrat non passivé, les signaux dus aux interactions des zones sensibles du substrat avec la vapeur d'eau présente dans les deux types d'échantillon couvre quasi complètement les signaux dus aux interactions de ces mêmes zones sensibles avec les gaz de combustion (environ 260 ppm de COV) en sorte qu'en données normalisées, la présence des gaz de combustion n'est quasiment pas visible (figure 16B).

Par contre, pour un substrat passivé avec un composé perfluoré, la vapeur d'eau présente dans les deux types d'échantillon génère une réponse moindre des zones sensibles et les résultats en données normalisées sont clairement différents pour l'échantillon de vapeur d'eau et l'échantillon de gaz de combustion (figure 17B). Les signaux dus aux composants du gaz de combustion autres que la vapeur d'eau sont bien visibles et détectables.

Cela illustre la capacité d'un revêtement perfluoré, lorsqu'il est utilisé pour passiver un substrat, à diminuer les interférences avec l'humidité qui est présente dans de nombreux échantillons odorants souvent à des concentrations bien plus élevées que celles des composés que l'on cherche à détecter et identifier.

**RÉFÉRENCES CITÉES**

**[1]** S. Brenet et al., ISOCS/IEEE International Symposium on Olfaction and Electronic Nose (ISOEN), IEEE, 2017, pp. 1-3

**[2]** Y. C. Lee et al., 19th International Conference on Solid-State Sensors, Actuators and Microsystems (Transducers), IEEE, 2017, pp. 672-675

**[3]** Handbook of Machine Olfaction: Electronic Nose Technology, chapitre 13, John Wiley & Sons 2006

**[4]** S. Di Carlo et M. Falasconi, Drift correction methods for gas chemical sensors in artificial olfaction systems: techniques and challenges. InTech: 2012

**[5]** K. Arshak, E. Moore, G. M. Lyons, J. Harris, and S. Clifford, A review of gas sensors employed in electronic nose applications, Sensor Review 2004, vol. 24, pp. 181-198

**[6]** T. Wasilewski, J. Gȩbicki, and W. Kamysz, *Bioelectronic nose: Current status and perspectives, Biosensors and Bioelectronics* 2017, vol. 87, pp. 480-494

**[7]** A. D. Wilson et M. Baietto, Applications and Advances in Electronic-Nose Technologies, Sensors (Basel) 2009, vol. 9, pp. 5099-5148

**Revendications**

1. Système de détection d'un nez électronique apte à détecter et identifier un ensemble E de composés susceptibles d'être présents dans un échantillon gazeux, lequel système de détection comprend une pluralité de capteurs de détection à réactivité croisée pour fournir des signaux représentatifs de la présence d'un ou plusieurs composés de l'ensemble E dans l'échantillon gazeux, chaque capteur de détection comprenant une partie sensible, et est **caractérisé en ce qu'**il comprend :

   - au moins un capteur de référence pour fournir un signal représentatif du bruit de mesure du système de détection, le capteur de référence comprenant une partie sensible ;
   - un substrat comprenant une surface sur laquelle sont disposées les parties sensibles des capteurs de détection et la partie sensible du capteur de référence ;

et **en ce que** la surface du substrat comprend :

* une pluralité de zones sensibles, chacune de ces zones sensibles correspondant à la partie sensible de l'un des capteurs de détection et comprenant au moins un récepteur capable d'interagir physico-chimiquement avec au moins un composé de l'ensemble E, et
* au moins une zone sensible qui correspond à la partie sensible du capteur de référence et qui est fonctionnalisée avec au moins un composé fluoré choisi parmi les composés comprenant au moins un groupe alkyle terminal perfluoré et les polymères fluorés.

2. Système de détection selon la revendication 1, **caractérisé en ce que** la surface du substrat est une surface passivée.

3. Système de détection selon la revendication 2, **caractérisé en ce que** la surface du substrat est passivée avec un composé fluoré choisi les composés comprenant au moins un groupe alkyle terminal perfluoré et les polymères fluorés.

4. Système de détection selon l'une quelconque des revendications 1 à 3, , **caractérisé en ce que** le composé fluoré est choisi parmi les composés de formule $C_vF_{2v+2}$ dans laquelle v est un entier allant de 4 à 20, et les composés de formule $C_wF_{2w+1}$-$(L)_x$-Z dans laquelle w est un entier allant de 1 à 12, x vaut 0 ou 1, L représente un groupe divalent espaceur, tandis que Z représente un groupe apte à permettre la fixation du composé sur la surface du substrat, de préférence un groupe thiol, amine, silanol, carbonyle ou carboxyle.

5. Système de détection selon la revendication 4, **caractérisé en ce que** le groupe divalent espaceur est un groupe hydrocarboné linéaire ou ramifié, saturé ou insaturé, comprenant de 1 à 20 atomes de carbone et éventuellement un ou plusieurs hétéroatomes.

6. Système de détection selon la revendication 5, **caractérisé en ce que** le groupe divalent espaceur est un groupe alkylène divalent comprenant de 1 à 20 atomes de carbone, de préférence de 1 à 12 atomes de carbone.

7. Système de détection selon l'une quelconque des revendications 4 à 6, **caractérisé en ce que** le composé fluoré est un perfluoralcanethiol de formule $CF_3(CF_2)_y(CH_2)_zSH$ dans laquelle y est un nombre entier allant de 0 à 11 et z est un nombre entier allant de 0 à 20, de préférence de 1 à 12.

8. Système de détection selon la revendication 7, **caractérisé en ce que** le composé fluoré est le 1*H*,1*H*-trifluoroéthanethiol, le 1*H*,1*H*,2*H*,2*H*-perfluoro-pentanethiol, le 1*H*,1*H*,2*H*,2*H*-perfluorohexanethiol, le 1*H*,1*H*,2*H*,2*H*-perfluorooctanethiol ou le 1*H*,1*H*,2*H*,2*H*-perfluorodécanethiol.

9. Système de détection selon l'une quelconque des revendications 1 à 3, **caractérisé en ce que** le composé fluoré est choisi parmi les polytétrafluoroéthylènes, les fluorures de polyvinyle, les fluorures de polyvinylidène, les perfluoroalcoxy alcanes, les copolymères d'éthylène et de propylène fluorés et les poly(éthylène-co-tétrafluoroéthylène).

10. Système de détection selon la revendication 1, **caractérisé en ce que** la zone sensible correspondant à la partie sensible du capteur de référence est formée d'une couche auto-assemblée du composé fluoré.

11. Système de détection selon l'une quelconque des revendications 1 à 10, **caractérisé en ce que** les capteurs de détection et le capteur de référence sont des capteurs résistifs, piézo-électriques, mécaniques, acoustiques et/ou optiques.

12. Système de détection selon la revendication 11, **caractérisé en ce que** les capteurs de détection et le capteur de référence sont des capteurs optiques à résonance des plasmons de surface.

13. Nez électronique apte à détecter et identifier un ensemble E de composés susceptibles d'être présents dans un échantillon gazeux, **caractérisé en ce qu'**il comprend un système de détection tel que défini dans l'une quelconque des revendications 1 à 12.

14. Nez électronique selon la revendication 13, **caractérisé en ce que** les composés de l'ensemble E sont des composés organiques volatils, le sulfure d'hydrogène et l'ammoniac.

**Patentansprüche**

1. Detektionssystem für eine elektronische Nase, die dazu geeignet ist, eine Menge E von Verbindungen, die in einer gasförmigen Probe vorhanden sein können, zu detektieren und zu identifizieren, wobei das Detektionssystem eine Vielzahl von kreuzreaktiven Detektionssensoren zum Bereitstellen von Signalen enthält, die für das Vorhandensein von einer oder mehreren Verbindungen der Menge E in der gasförmigen Probe repräsentativ sind, wobei jeder Detektionssensor einen empfindlichen Teil umfasst,

**dadurch gekennzeichnet ist, dass** es enthält:

- zumindest einen Referenzsensor zur Bereitstellung eines für das Messrauschen des Detektionssystems repräsentativen Signals, wobei der Referenzsensor einen empfindlichen Teil enthält;
- ein Substrat mit einer Oberfläche, auf der die empfindlichen Teile der Detektionssensoren und der empfindliche Teil des Referenzsensors angeordnet sind;

und dass die Oberfläche des Substrats enthält:

* eine Vielzahl von empfindlichen Bereichen, wobei jeder dieser empfindlichen Bereiche dem empfindlichen Teil eines der Detektionssensoren entspricht und zumindest einen Rezeptor enthält, der in der Lage ist, mit zumindest einer Verbindung der Menge E physikalisch-chemisch zu interagieren, und
* zumindest einen empfindlichen Bereich, der dem empfindlichen Teil des Referenzsensors entspricht und der mit zumindest einer fluorierten Verbindung funktionalisiert ist, die aus den Verbindungen mit zumindest einer endständigen Perfluoralkylgruppe und den Fluorpolymeren ausgewählt ist.

2. Detektionssystem nach Anspruch 1, **dadurch gekennzeichnet, dass** die Oberfläche des Substrats eine passivierte Oberfläche ist.

3. Detektionssystem nach Anspruch 2, **dadurch gekennzeichnet, dass** die Oberfläche des Substrats mit einer fluorierten Verbindung passiviert ist, die aus Verbindungen mit zumindest einer endständigen Perfluoralkylgruppe und Fluorpolymeren ausgewählt ist.

4. Detektionssystem nach einem der Ansprüche 1 bis 3, **dadurch gekennzeichnet, dass** die fluorierte Verbindung ausgewählt ist aus Verbindungen der Formel $C_vF_{2v+2}$, worin v eine ganze Zahl von 4 bis 20 ist, und Verbindungen der Formel $C_wF_{2w+i}-(L)_x-Z$, worin w eine ganze Zahl von 1 bis 12 ist, x 0 oder 1 ist, L eine zweiwertige Spacergruppe darstellt, während Z eine Gruppe darstellt, die die Bindung der Verbindung an die Oberfläche des Substrats ermöglichen kann, vorzugsweise eine Thiol-, Amino-, Silanol-, Carbonyl- oder Carboxylgruppe.

5. Detektionssystem nach Anspruch 4, **dadurch gekennzeichnet, dass** die zweiwertige Spacergruppe eine lineare oder verzweigte, gesättigte oder ungesättigte Kohlenwasserstoffgruppe ist, die 1 bis 20 Kohlenstoffatome und gegebenenfalls ein oder mehrere Heteroatome umfasst.

6. Detektionssystem nach Anspruch 5, **dadurch gekennzeichnet, dass** die zweiwertige Spacergruppe eine zweiwertige Alkylengruppe ist, die 1 bis 20 Kohlenstoffatome, vorzugsweise 1 bis 12 Kohlenstoffatome, umfasst.

7. Detektionssystem nach einem der Ansprüche 4 bis 6, **dadurch gekennzeichnet, dass** die fluorierte Verbindung ein Perfluoralkanthiol der Formel $CF_3(CF_2)_y(CH_2)_zSH$ ist, worin y eine ganze Zahl von 0 bis 11 und z eine ganze Zahl von 0 bis 20, vorzugsweise von 1 bis 12, ist.

8. Detektionssystem nach Anspruch 7, **dadurch gekennzeichnet, dass** die fluorierte Verbindung 1*H*,1*H*-Trifluorethanthiol, 1*H*,1*H*,2*H*,2*H*-Perfluorpentanthiol, 1*H*,1*H*,2*H*,2*H*-Perfluorhexanthiol, 1*H*,1*H*,2*H*,2*H*-Perfluoroctanthiol oder 1*H*,1*H*,2*H*,2*H*-Perfluorodecanthiol ist.

9. Detektionssystem nach einem der Ansprüche 1 bis 3, **dadurch gekennzeichnet, dass** die fluorierte Verbindung ausgewählt ist aus Polytetrafluorethylenen, Polyvinylfluoriden, Polyvinylidenfluoriden, Perfluoralkoxyalkanen, Copolymeren von fluoriertem Ethylen und Propylen und Poly(ethylen-co-tetrafluorethylen).

10. Detektionssystem nach Anspruch 1, **dadurch gekennzeichnet, dass** der empfindliche Bereich, der dem empfindlichen Teil des Referenzsensors entspricht, aus einer selbstorganisierten Schicht der fluorierten Verbindung gebildet ist.

11. Detektionssystem nach einem der Ansprüche 1 bis 10, **dadurch gekennzeichnet, dass** die Detektionssensoren und der Referenzsensor resistive, piezoelektrische, mechanische, akustische und/oder optische Sensoren sind.

12. Detektionssystem nach Anspruch 11, **dadurch gekennzeichnet, dass** die Detektionssensoren und der Referenzsensor optische Sensoren mit Oberflächenplasmonenresonanz sind.

13. Elektronische Nase, die dazu geeignet ist, eine Menge E von Verbindungen, die in einer gasförmigen Probe vorhanden sein können, zu detektieren und zu identifizieren, **dadurch gekennzeichnet, dass** sie ein Detektionssystem nach einem der Ansprüche 1 bis 12 enthält.

14. Elektronische Nase nach Anspruch 13, **dadurch gekennzeichnet, dass** die Verbindungen der Menge E flüchtige organische Verbindungen, Schwefelwasserstoff und Ammoniak sind.

**Claims**

1. Detection system for an electronic nose capable of detecting and identifying a set E of compounds likely to be present in a gas sample, said detection system comprises a plurality of detection sensors having cross-reactivity to provide signals representing the presence of one or more compounds of set E in the gas sample, each detection sensor comprising a sensitive portion, and is **characterized in that** it comprises:

    - at least one reference sensor to provide a signal representing the measurement noise of the detection system, the reference sensor comprising a sensitive portion;
    - a substrate comprising a surface on which there are arranged the sensitive portions of the detection sensors and the sensitive portion of the reference sensor;

    and **in that** the surface of the substrate comprises:

    * a plurality of sensitive zones, each of these sensitive zones corresponding to the sensitive portion of one of the detection sensors and comprising at least one receptor capable of interacting physicochemically with at least one compound of set E; and
    * at least one sensitive zone which corresponds to the sensitive portion of the reference sensor and which is functionalised with at least one fluorinated compound selected from among compounds comprising at least one terminal perfluorinated alkyl group and fluorinated polymers.

2. Detection system according to claim 1, **characterized in that** the surface of the substrate is a passivated surface.

3. Detection system according to claim 2, **characterized in that** the substrate is passivated with a fluorinated compound selected from among compounds comprising at least one terminal perfluorinated alkyl group and fluorinated polymers.

4. Detection system according to any of claims 1 to 3, **characterized in that** the fluorinated compound is selected from among the compounds of formula $C_vF_{2v+2}$ where v is in an integer ranging from 4 to 20, and the compounds of formula $C_wF_{2w+1}$-$(L)_x$-Z where w is an integer ranging from 1 to 12, x is 0 or 1, L is a divalent spacer group, whilst Z is a group able to allow the fixing of the compound on the surface of the substrate, preferably a thiol, amine, silanol, carbonyl or carboxyl group.

5. Detection system according to claim 4, **characterized in that** the divalent spacer group is a linear or branched, saturated or unsaturated hydrocarbon group having 1 to 20 carbon atoms and optionally one or more heteroatoms.

6. Detection system according to claim 5, **characterized in that** the divalent spacer group is a divalent alkylene group having 1 to 20 carbon atoms, preferably 1 to 12 carbon atoms.

7. Detection system according to any of claims 4 to 6, **characterized in that** the fluorinated compound is a perfluoroalkanethiol of formula $CF_3(CF_2)_y(CH_2)_zSH$ where y is an integer ranging from 0 to 11 and z is an integer ranging from 0 to 20, preferably from 1 to 12.

8. Detection system according to claim 7, **characterized in that** the fluorinated compound is 1*H*,1*H*-trifluoroethanethiol, 1*H*,1*H*,2*H*,2*H*-perfluoropentanethiol, 1*H*,1*H*,2*H*,2*H*-perfluorohexanethiol, 1*H*,1*H*,2*H*,2*H*-perfluorooctanethiol or *1H,1H,2H,2H*-perfluorodecanethiol.

9. Detection system according to any of claims 1 to 3, **characterized in that** the fluorinated compound is selected from among polytetrafluoroethylenes, polyvinyl fluorides, polyvinylidene fluorides, perfluoroalkoxy alkanes, fluorinated ethylene and propylene copolymers, and poly(ethylene-*co*-tetrafluoroethylenes).

10. Detection system according to claim 1, **characterized in that** the sensitive zone corresponding to the sensitive portion of the reference sensor is formed of a self-assembled layer of the fluorinated compound.

11. Detection system according to any of claims 1 to 10, **characterized in that** the detection sensors and the reference sensor are resistive, piezoelectric, mechanical, acoustic and/or optical sensors.

12. Detection system according to claim 11, **characterized in that** the detection sensors and the reference sensor are surface plasmon resonance optical sensors.

13. Electronic nose capable of detecting and identifying a set E of compounds likely to be present in a gas sample, **characterized in that** it comprises a detection system as defined in any of claims 1 to 12.

14. Electronic nose according to claim 13, **characterized in that** the compounds of set E are volatile organic compounds, hydrogen sulfide and ammonia.

D1   D2   D3   D4

10

11

D5   D6   D7   R1

FIG. 1

D1   D2   D3   D4

10

11

D5   D6   D7   R1

FIG. 2

D1   D2   D3   D4

10

11

D5   D6   D7   R1

FIG. 3

FIG. 4

FIG. 5

FIG. 6

FIG. 7

21

Δ%R

FIG. 8A

Δ%R

FIG. 8B

FIG. 9

FIG. 10

FIG. 11A

FIG. 11B

FIG. 12A

FIG. 12B

FIG. 13A

FIG. 13B

FIG. 14A

FIG. 14B

FIG. 15

EP 3 665 478 B1

FIG. 16A

FIG. 16B

FIG. 17A

FIG. 17B

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- au 17ème Symposium International sur l'Olfaction et les Nez électroniques (28-31 mai 2017, Montréal, Canada). **S. BRENET et al.** 2017 SOCS/IEEE International Symposium on Olfaction and Electronic Nose (ISOEN). IEEE, 2017, 1-3
- **Y. C. LEE et al.** 19th International Conference on Solid-State Sensors, Actuators and Microsystems (Transducers). IEEE, 2017, 672-675
- Handbook of Machine Olfaction: Electronic Nose Technology. John Wiley & Sons, 2006
- **S. DI CARLO ; M. FALASCONI.** *Drift correction methods for gas chemical sensors in artificial olfaction systems: techniques and challenges,* 2012
- **K. ARSHAK et al.** *Sensor Review,* 2004, vol. 24, 181-198

- **T. WASILEWSKI et al.** *Biosensors and Bioelectronics,* 2017, vol. 87, 480-494
- **A. D. WILSON ; M. BAIETTO.** *Sensors (Basel),* 2009, vol. 9, 5099-5148
- **S. BRENET et al.** ISOCS/IEEE International Symposium on Olfaction and Electronic Nose (ISOEN). IEEE, 2017, 1-3
- **K. ARSHAK ; E. MOORE ; G. M. LYONS ; J. HARRIS ; S. CLIFFORD.** *A review of gas sensors employed in electronic nose applications, Sensor Review,* 2004, vol. 24, 181-198
- **A. D. WILSON ; M. BAIETTO.** *Applications and Advances in Electronic-Nose Technologies, Sensors (Basel),* 2009, vol. 9, 5099-5148